# EUROPEAN PATENT SPECIFICATION

(11) **EP 0 274 489 B1**
(45) Date of publication and mention of the grant of the patent: **15.10.1997**
(21) Application number: 87904286.9
(22) Date of filing: 30.06.1987
(51) Int. Cl.: C12N 15/00, C12P 21/00, C12N 5/00, A01K 67/00

(54) **PEPTIDE PRODUCTION**
HERSTELLUNG VON PEPTIDEN
PRODUCTION DE PEPTIDES

(30) Priority: 30.06.1986 GB 8615942
(43) Date of publication of application: 20.07.1988
(60) Divisional application: 97200314.9
(73) Proprietor: PPL THERAPEUTICS (SCOTLAND) LIMITED, Edinburgh EH25 9PP (GB)
(72) Inventor: CLARK, Anthony, John, Edinburgh (GB); LATHE, Richard, F-67100 Strasbourg (FR)
(74) Representative: Sheard, Andrew Gregory
(86) International application number: GB8700458
(87) International publication number: WO8800239

(56) References cited:
- EP-A- 0 264 166
- Nature, Volume 315, 20 June 1985, R.E. HAMMER et al.: "Production of Transgenic Rabitts, Sheep and Pips by Microinjection", pages 680-683 see the whole document cited in the application
- Trends in Biotechnology, Volume 5, No. 1, January 1987, Elevier Science Publishers B.V., (Amsterdam, NL), A.J. CLARK et al.: "Pharmaceuticals from Transgenic Livestock", pages 20-24 see the whole document

## Description

This invention relates to a method of producing a substance comprising a polypeptide. More particularly, the invention relates to protein production and to the production of biological materials whose formation is catalysed by enzymic proteins.

Recombinant DNA technology has been used increasingly over the past decade for the production of commercially important biological materials. To this end, the DNA sequences encoding a variety of medically important human proteins have been cloned. These include insulin, plasminogen activator, alpha₁-antitrypsin and coagulation factors VIII and IX. At present, even with the emergent recombinant DNA techniques, these proteins are usually purified from blood and tissue, an expensive and time consuming process which may carry the risk of transmitting infectious agents such as those causing AIDS and hepatitis.

Although the expression of DNA sequences in bacteria to produce the desired medically important protein looks an attractive proposition, in practice the bacteria often prove unsatisfactory as hosts because in the bacterial cell foreign proteins are unstable and are not processed correctly.

Recognizing this problem, the expression of cloned genes in mammalian tissue culture has been attempted and has in some instances proved a viable strategy. However batch fermentation of animal cells is an expensive and technically demanding process.

There is therefore a need for a high yield, low cost process for the production of biological substances such as correctly modified eukaryotic polypeptides. The absence of agents that are infectious to humans would be an advantage in such a process.

EP-A-0264166, published on 20 April 1988 and claiming a priority date of 9 April 1986, relates to certain transgenic animals secreting desired proteins into milk and discloses as a preferred embodiment the use of the whey acidic protein ("WAP") promoter.

According to a first aspect of the present invention, there is provided a method of producing a substance comprising a polypeptide, the method comprising:
(a) producing milk in the mammary gland of an adult, female, non-human mammal whose genetic material includes a genetic construct comprising:
   (i) a DNA sequence coding for the polypeptide; and
   (ii) a DNA sequence from a β-lactoglobulin gene to direct expression of the DNA sequence coding for the polypeptide; and
(b) collecting the milk produced in step (a). The substance will generally be recovered from milk of the adult female mammal, either before or preferably after post-translational modification.

As used in this specification, the term "polypeptide" refers to a molecule comprising a chain of amino acids, whether sufficiently long or not to be properly classed as a protein. The polypeptide is preferably sufficiently long to be a protein. The "substance comprising a polypeptide" may be the polypeptide itself, or it may be a modified (for example, glycosylated) polypeptide. Alternatively, or in addition, the substance can be cross linked, by post-translational modification of a polypeptide.

The present invention may be used to produce, for example, peptide hormones, blood coagulation factors (particularly factors VIII and IX) or subunits of them (particularly factors VIII and IX), blood proteins (for example beta-globin) and serum proteins (for example alpha₁-antitrypsin) proteins for foodstuffs, including natural or altered milk proteins of the host mammal, or enzymes.

Enzymes produced by the present invention may be able to act on their substrates in situ in the mammary gland. The present invention encompasses in a second aspect a method of producing a substance which is the reaction product of an enzyme, the method comprising:
(a) producing milk in the mammary gland of an adult, female, non-human mammal whose genetic material includes a genetic construct comprising:
   (i) a DNA sequence coding for the enzyme; and
   (ii) a DNA sequence from a milk whey protein gene to direct expression of the DNA sequence coding for the enzyme; and
(b) allowing the enzyme to catalyse the formation of the reaction production from one or more substrates of the enzyme.
The reaction product will generally be recovered from milk of the adult female mammal. The preferred milk whey protein gene is a β-lactoglobulin gene.

According to a third aspect of the invention, there is provided a process for the production of a transgenic mammal, the process comprising:
(a) incorporating *in vitro* in a genetic construct comprising:
   (i) a DNA sequence coding for a polypeptide; and
   (ii) a DNA sequence from a β-lactoglobulin gene to direct expression of the DNA sequence coding for the polypeptide in the mammary gland of an adult, female mammal;
(b) incorporating the genetic construct into a non-human mammal's germline by injection into a fertilised egg or a cell of an embryo of the non-human mammal; and
(c) allowing the injected fertilised egg or embryo to develop into an adult mammal; and
(d) optionally breeding a female mammal, whose germline contains the genetic construct from the mammal produced in step (c).

The DNA sequence coding for the polypeptide ("the DNA sequence of interest") is preferably incorporated in vitro into a milk whey protein gene which is expressible in the mammary gland of an adult female mammal to form a fusion gene and the fusion gene is incorporated into the germline by injection into a fertilized egg of the mammal, whereafter the injected fertilized egg is allowed to develop into an adult female mammal. It is to be expected that not all the injected eggs will develop into adult females expressing the DNA sequence of interest. Apart from anything else approximately half the animals will be male, from which females can be bred in the following generations.

The relevant techniques whereby foreign DNA sequences can be introduced into the mammalian germ line have been developed in the mouse. At present the most efficient route entails the direct microinjection of a few hundred linear molecules of DNA into a pro-nucleus of a fertilized one cell egg, and this is the method of preference in the present invention. Microinjected eggs may then subsequently be transferred into the oviducts of pseudopregnant foster mothers and allowed to develop. It has been reported by Brinster et al in Proc Natl Acad Sci 82 (1985) 4438-4442 that about 25% of the mice that develop inherit one or more copies of the microinjected DNA. From a commercial point of view, it is clearly preferable to use as the host mammal in the present invention a species that is likely to have a larger milk yield. For this reason, domestic livestock animals such as sheep, pigs and cattle are preferred, as will be discussed in more detail below.

Working with large domestic animals requires a considerable investment, particularly in the number of animals required and their cost. While it is normal to obtain up to 30 eggs per superovulated mouse, a ewe gives only three to five. A further problem encountered with farm animals is that the eggs are opaque due to the presence of numerous vesicles, and this makes the identification and successful injection of pronuclei more difficult. Nevertheless, microinjection of DNA into eggs of rabbits, sheep and pigs has been reported by Hammer et al, Nature 315 (1985) 680-683, who inserted a metallothionein-growth hormone fusion gene into the germ line of all three mammals.

In the pig, 2,035 eggs were injected and transferred; 192 piglets were born, of which 20 carried the fusion gene. The results were not so encouraging in sheep; 1,032 eggs were injected and transferred, but 73 lambs were born of which only one contained the foreign DNA sequence.

Once integrated into the germ line, the foreign DNA may be expressed in the tissue of choice at high levels to produce a functional protein which can readily be harvested from the animal. To accomplish this, the DNA sequence coding for the peptide of interest will generally be fused to a DNA sequence or sequences that will mediate its expression in a suitable tissue. Although some initial experiments in mice show that tissue specific expression of inserted DNA to be somewhat variable (for example, Lacey et al, Cell 34 (1983) 343-356), the general concensus now is that correct tissue specific expression of most transfected genes is achievable. See Brinster et al Cell 27 (1981) 223-231; Swift et al Cell 38 (1984) 639-646; Shani Nature 314 (1985) 283-286; and Magram et al Nature 315 (1985) 338-340. For correct tissue specificity, it does appear to be important to remove all the prokaryote vector sequences, which are used in the cloning of DNA sequence of interest, prior to microinjection (Krumlauf et al Mol. Cell Biol. 5 (1985) 1639-1648).

While these publications do indicate that certain different tissues can be targeted in the construction of a transgenic animal, there is no suggestion of the desirability of any particular tissue for commercial production of polypeptide-containing substances. Neither do they suggest any particular gene which is expressed in a given tissue and which would serve to target the expression of the inserted DNA.

A number of factors must be taken into consideration with regard to the substance comprising the peptide and the tissue to which its expression is to be directed. Many proteins require extensive post-translational modification in order to exhibit full biological activity. For example, factor IX requires gamma-carboxylation of a specific subset of glutamic acid residues for biological activity (De Scipio and Davie, Biochemistry 18 899-904 (1979)). Liver, which is the site of natural synthesis of factor IX, is proficient in performing this modification. Fibroblasts are capable of carrying out gamma carboxylation of factor IX, although less efficiently: de la Salle et al Nature 316 268-270 (1985). However, certain proteins may be correctly modified only if synthesised in a specific tissue and, in some cases, it may be necessary to tailor the site of expression to the requirements of the proteins produced. It is believed that the use of the mammary gland as a tissue for expression overcomes, either wholly or to a satisfactory degree, this potential source of difficulty.

Harvesting from body fluids as opposed to solid tissue is desirable, because such routes are by and large renewable, and most proteins of biomedical importance are themselves secreted into body fluids. Secretion into the bloodstream is a possible route, either from liver or B lymphocytes, but the coagulant properties of blood and the presence of biologically active peptides and antigenic molecules may prove a hindrance to subsequent downstream processing.

The above difficulties may be overcome in accordance with the present invention by the use of the mammary gland as a tissue of expression. Milk is readily collected, available in large quantities and well characterized biochemically. Further, the major milk proteins are present in milk at high concentrations (from about 1 to 15 g/l).

There are four species of common farm animal in many countries of the world: pigs, goats, sheep and cattle, that is to say members of the family Suidae, the genus Capra, the genus Ovis and the genus Bos. Domesticated cattle are generally of the species Taurus.

While the present invention is not in its broadest aspect restricted to any particular species, genus or family of mammals, pigs, goats, sheep and cattle are preferred. Each has advantages and disadvantages in the context of the present invention. Some of these considerations stem from the fact that, according to a preferred aspect of the present invention, the DNA sequence coding for the polypeptide is incorporated in vitro into a whey protein gene which is expressible in the mammary gland of an adult female mammal to form a fusion gene and the fusion gene is injected into a fertilized egg of the mammal, whereafter the injected fertilized egg is allowed to develop into an adult female mammal.

Although the high milk yield obtained from cattle is a factor tending to suggest that the use of cattle would be preferred, the technical difficulties in manipulating the bovine embryo are greater than in the case of sheep, for example. Furthermore, the sheer cost of experiments involving cattle mean that the other three preferred species of domestic farm animals are the ones of choice.

Table 1 below shows how pigs, sheep and cattle compare with each other for the purposes of use in the present invention.

**TABLE 1**

| Comparison of Different Livestock for Embryo Manipulation and Milk Production. | | | |
|---|---|---|---|
| | PIG | SHEEP | CATTLE |
| Possibility of controlling time of follicile maturation | Yes | (Yes) | (No) |
| No. of oocytes per animal; no superovulation | 10 | 1 to 3 | 1 |
| No. of oocytes per animal; superovulated | 15 to 20 | 4 to 10 | possibly 6 |
| Visualisation of pronuclei | (Yes) | (Yes) | (No) |
| Seasonal breeding | No | Yes | No |
| Relative cost per embryo transfer, including nominal cost of animals, per embryo | 1.0 | 1.8 | 110 |
| Milk yield, litres/day at peak production | n.a. | 1 to 3* | 5 to 30* |

| | | | |
|---|---|---|---|
| * Depending on breed. n.a. = data not available | | | |

The significance of the visualisability of the pronuclei is that it is a preferred feature of the present invention to inject the fusion gene containing the DNA sequence coding for the peptide into the pronucleus of the fertilised egg.

Milk yield data for pigs are not not readily available, but in sheep, the rate of milk production falls within the range of from 1 to 3 litres per day, depending on the breed. It is of note that specialized equipment for harvesting milk from sheep is available from commercial suppliers, as of course it is for cattle. Sheep are therefore the animals of choice, in particular dairy sheep such as East Frieslands. A strain obtained by crossing a suitable high milk producing line within Blackface sheep is seen as a viable alternative.

The lactating mammary gland is a highly specialized organ comprising an extensive sytem of ducts that drain complex lobules of secretory cells. Mammary cells are adapted in many ways to high rates of secretion. For example, they have specialized transport mechanisms that ensure the efficient uptake of precursors from the blood, and an extensive system of intracellular membranes (rough endoplasmic reticulum, Golgi apparatus etc) that enable high rates of protein synthesis, post-translational modification and export from the cell.

The mammary gland is dependent on hormones for all aspects of its growth and function. Review articles on the mammary gland include those by Topper and Freeman, Physiol Rev 60 1049-1106 (1980) and Forsyth in "The Biochemistry of Lactation", Mepham (Ed), Elsevier (1982) 309-349. The hormones affecting the mammary gland mediate the striking changes that occur in the gland during pregnancy and lactation. In the ewe, for example, this leads to a near-doubling in total cell number as well as to changes in the proportions of the various cell types and to the terminal differentiation of the secretory cells.

The mammary gland secretes a number of different proteins into the milk. There are qualitative and quantitative differences in the composition of milk from different species, although a general distinction can be made between the caseins and the soluble (whey) proteins (see Jenness in "Developments in Dairy Chemistry, I", Fox (Ed) Elsevier (1982) 87-109). The major ruminant whey proteins that are synthesised in the mammary gland are alpha-lactalbumin and beta-lactoglobulin.

There are three major types of casein. These are alpha-casein, beta-casein and kappa-casein and appear in most of the species characterized. In milk, they serve to sequester calcium with which they are aggregated in the form of micelles. The function of beta-lactoglobulin, the major whey protein in ruminants, is unknown, although it appears to interact with kappa-casein (Brunner, J. Dairy Sci. 64 1038-1054 (1981)). Alpha-lactalbumin is an essential cofactor in the conversion of glucose and galactose to lactose (Brew, Nature 223 671-672 (1969)). Table 2 shows the protein composition of various milks.

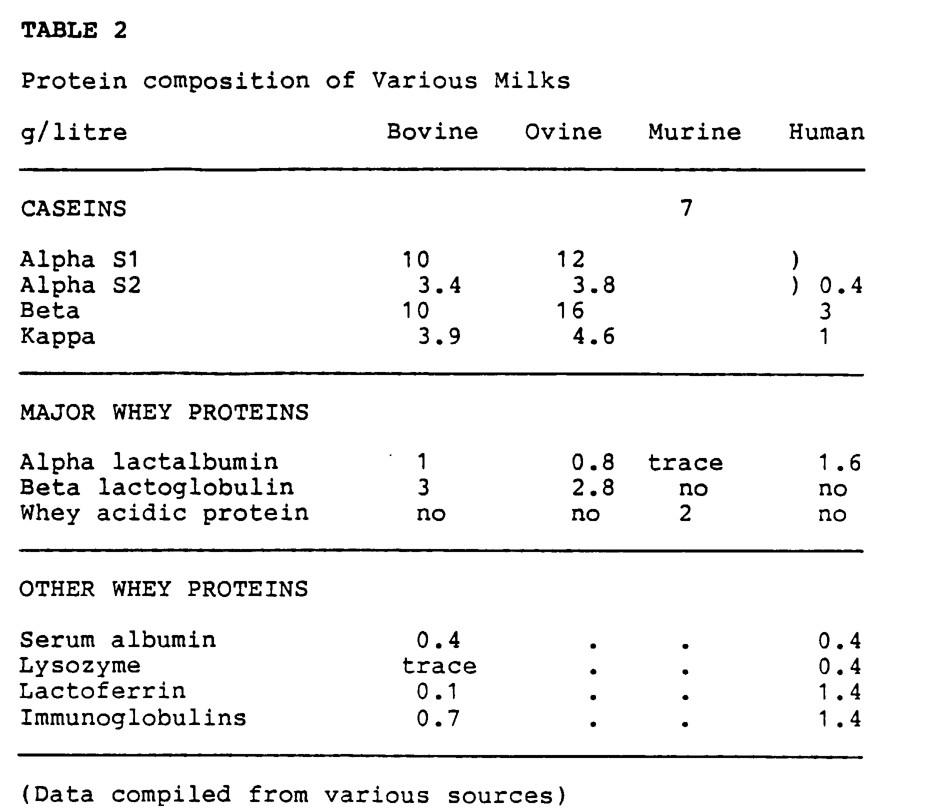

For the most part it is believed that the different milk proteins are encoded by single copy genes (Mercier and Gaye in "The Biochemistry of Lactation", Mepham (Ed) Elsevier (1983) 177-225). The casein genes appear to be linked both in the mouse (Rosen et al Biochem Soc Trans, 9 112 (1982)) and in the cow (Grossclaude Proc. 16th Intl. Conf. Animal Blood Groups Biochem. Polymorphy. 1 54-59 (1979)). Because the casein genes appear to be expressed at a far higher level than the whey protein genes, it would naturally be thought that the casein genes would be the genes of choice in which to incorporate the foreign DNA for expression. However, contrary to the general expectation, the present invention provides that the DNA sequence coding for the peptide of interest be incorporated into a gene coding for a whey protein.

The gene for rat alpha-lactalbumin comprises four exons and encompasses 2.5 kb of chromosomal DNA (Qasba and Safaya Nature **311** 377-380 (1984)). The gene for bovine alpha-lactalbumin appears to be similarly organised. It has now been discovered that ovine beta-lactoglobulin is most probably a single copy gene and comprises seven exons within a 4.9 kb transcription unit: this gene is the gene of preference for use in the present invention.

Changes in the levels of milk protein mRNAs during mammary development in the rat (Nakhasi and Qasba, J. Biol. Chem **254** 6016-6025 (1979)), rabbit (Shuster et al Eur. J. Biochem. **71** 193-199 (1976)) and mouse (Pauley et al Nature **275** 455-457 (1978)) have been assessed using both in vitro translation and cDNA hybridisation. The mammary gland accumulates some 80,000 to 100,000 molecules of milk protein-specific mRNAs resulting from a coordinated mechanism comprising both a high rate of milk protein mRNA synthesis and an efficient stabilisation of these molecules (Mercier and Gaye, op. Cit. and Guyette et al Cell **17** 1013-1073 (1979)). There are, however, exceptions. In the ewe beta-casein makes up 45% of the total milk protein, yet only 17% of cDNA clones isolated from a cDNA library corresponded to this gene (Mercier et al Biochimie 67 959-971 (1985)). In the rabbit, the amount of alpha-lactalbumin sequestered into microsomal vesicles was much lower than that expected from the rate of protein synthesis in the absence of microsomal vesicles, possibly suggesting that the signal peptide does not have an optimal configuration (Gaye et al Biochimie **64** 173-184 (1982)). The primary translation products of many of the milk protein genes are extensively modified. For example, alpha-lactalbumin is N-glycosylated, kappa-casein is O-glycosylated and alpha- and beta-casein are O-phosphorylated. In addition to its role in post-translational modification, the Golgi complex is also involved in condensing and packaging caseins into micelles. It is a possibility that the caseins and the whey proteins are exported from the cell by different routes. In this regard it is of interest to note that, in both inter- and intra-specific comparisons, the casein signal peptide (in comparison to a large proportion of the rest of the molecules) is highly conserved, suggesting that it may play a role in targeting the nascent peptide into the correct secretory pathway (Mepham et al op cit). As described above, milk protein genes are abundantly expressed in the lactating mammary gland. In the ewe, for example, alpha_{S1}-casein mRNA accounts for about 30%, and beta-lactoglobulin accounts for about 5%, of polyA+RNA (Mercier et al (1985) op cit). Given that these transcripts originate from single copy genes, then these levels indicate high rates of transcription and efficient mRNA stabilisation (Teyssot and Houdebine, Eur. J. Biochem. 110 263-272 (1980)). Specific mRNA stabilisation may be mediated by sequences present in the mature mRNA. In preferred embodiments of the present invention, similar levels of expression of the DNA sequence that is inserted into the ovine germ line may be achievable. This is accomplished by linking it to the DNA sequences associated with a milk whey protein gene that mediate the high levels of tissue-specific expression and mRNA stabilisation: beta-lactoglobulin is the gene of preference.

Transferring new genes into mice is now a routine procedure. In transgenic mice that exhibit high levels of tissue specific expression of a foreign gene, the exogenous DNA comprises not only the structural gene but also the 5' and 3' flanking sequences. Although there is an abundance of evidence suggesting that many important regulatory elements are located 5' to the mRNA cap site (see for example McKnight and Kingsbury, Science **217** 316-324 (1982); Payvar et al Cell 35 381-392 (1983); Renkawitz et al Cell 37 503-510 (1984); Karin et al Nature 308 513-518 (1984)) it is also evident that important regulatory sequences, particularly those mediating tissue-specific expression, may reside within the structural gene or even 3' to it (Charnay et al Cell 38 251-263 (1984); Gillies et al Cell 33 717-728 (1983)). Furthermore exonic sequences (i.e. those present in mature mRNA) may contain sequences that mediate mRNA stability. Table 3 illustrates some details of tissue-specific expression of foreign genes in mice.

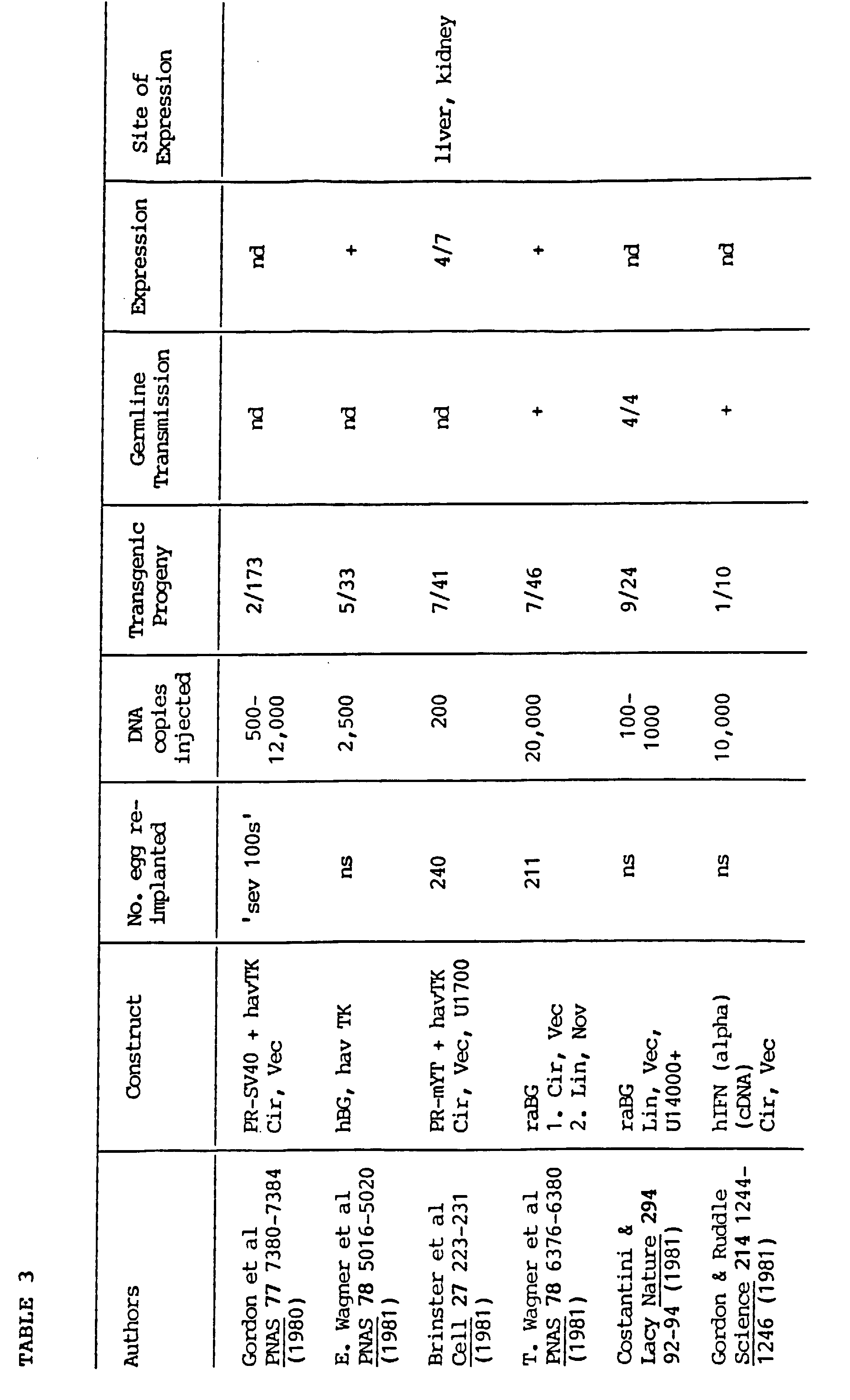

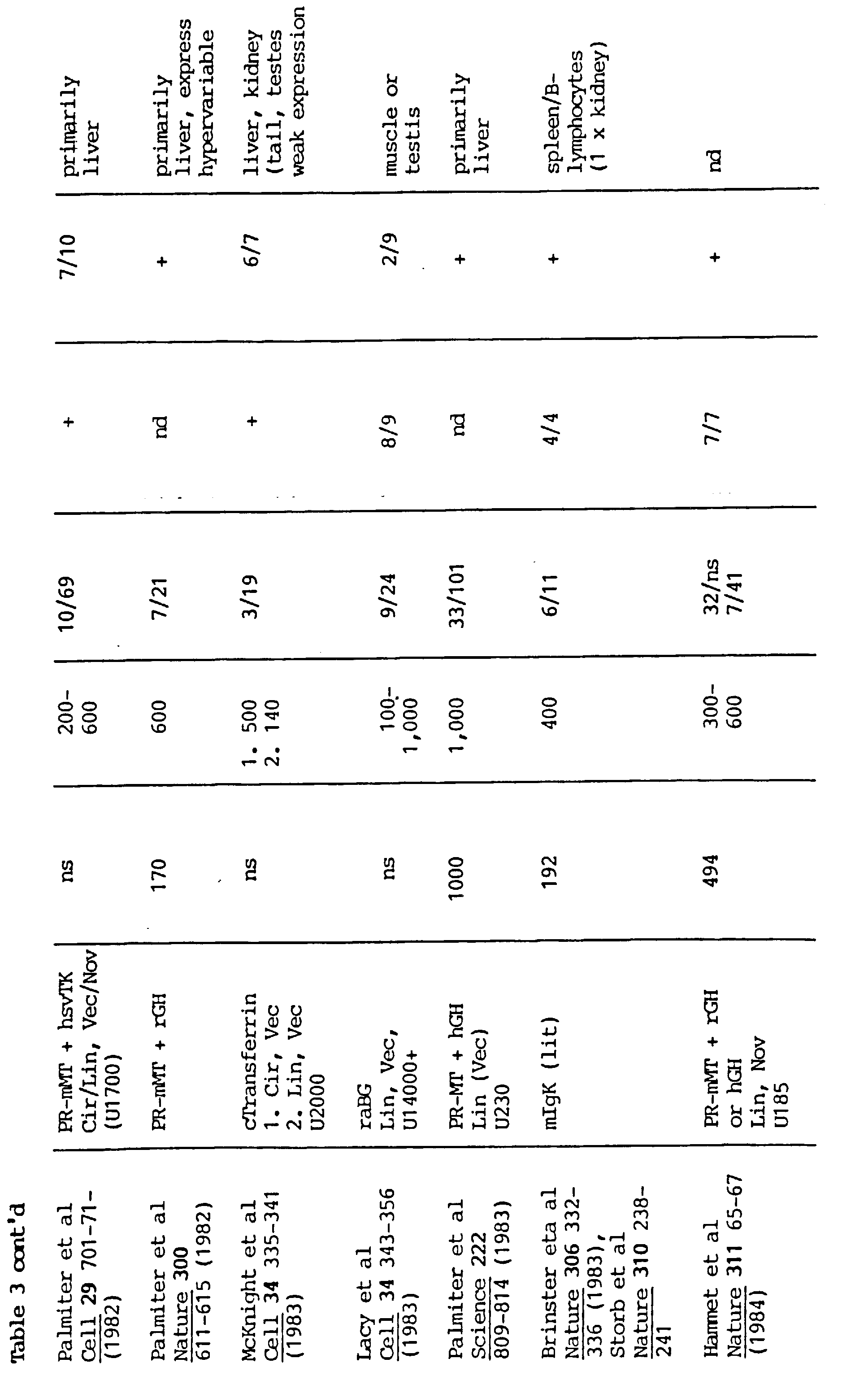

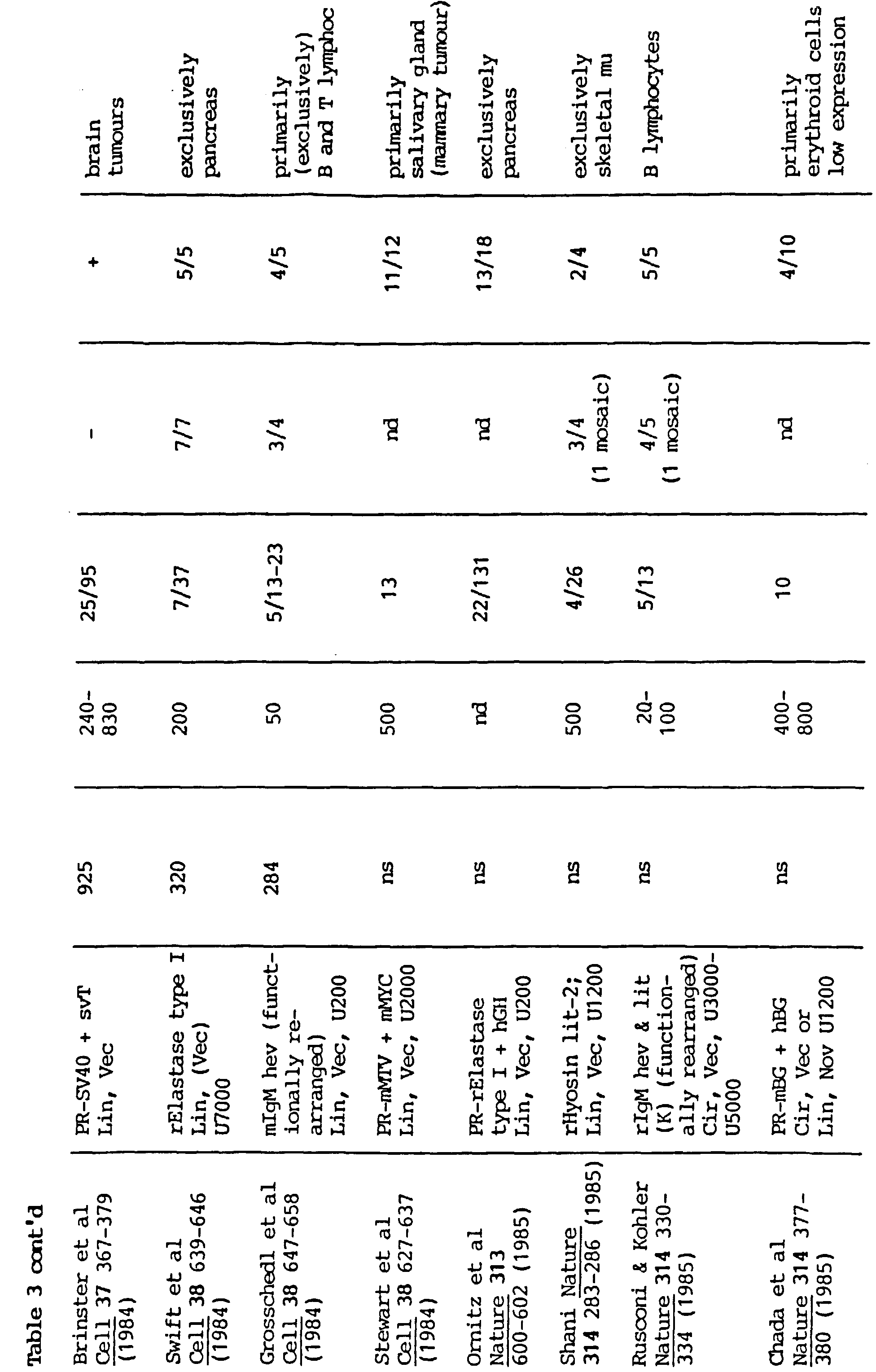

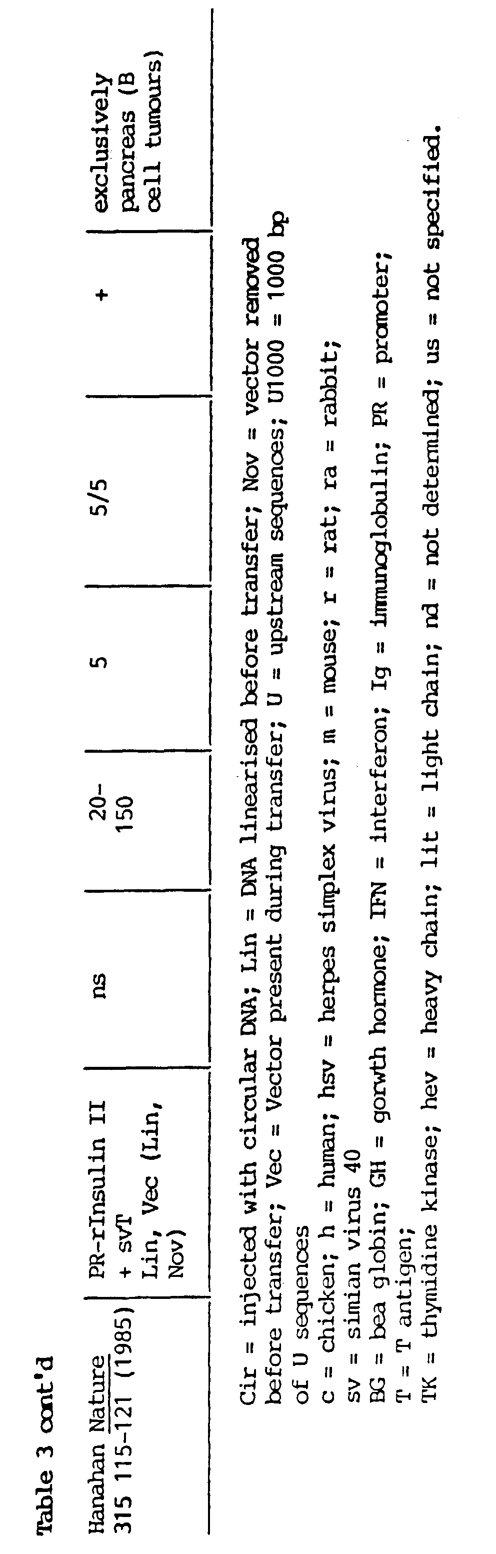

It is for the above reason that the above preferred fusion genes that will be used to direct the expression of the DNA sequence coding for the peptide of interest will comprise one or more of: a promoter; a start site for transcription; one or more (presumed) distal 5' regulatory sequences of a milk protein gene; structural milk protein gene sequences; 3' sequences flanking a milk protein gene. The most preferred fusion genes comprise sequences of all these types.

A fusion gene of choice will consist of a cDNA sequence coding for the peptide of interest inserted into the first exon of a whey protein gene. It is preferred that several kb of the 5' flanking sequences of the milk protein gene will be included in such a fusion gene. In these constructs, secretion of the peptide of interest will preferably be mediated by its own signal peptide; it is therefore preferred that the fusion gene contain a signal peptide for the peptide of interest. However, a tissue-specific signal peptide may be important for targeting the nascent peptide into the correct secretory pathway (as discussed above). Therefore, it is particularly preferred that DNA sequences encoding the signal peptide of the whey protein gene will be precisely fused to the DNA sequences of the insert that encode the N-terminal amino acid of the mature protein. The 3' end of the insert will preferably terminate after its stop codon, but before its own message cleavance and polyadenylation site. Downstream of the site of insertion, the rest of the structural gene will generally be retained, as well as some 3' flanking sequences.

In a transgenic animal, such a construct should maximise the chances of obtaining high levels of mammary gland-specific expression. The primary transcript should be correctly polyadenylated and spliced at the sites retained in the whey protein gene. The mature message should contain sequences for efficient stabilisation of mRNA. In constructs employing the milk gene signal peptide, the mature mRNA should be translated to yield a fusion pre-peptide in which the signal peptide derived from the milk protein gene efficiently directs the secretion of the mature peptide encoded by the cDNA insert.

Ovine beta-lactoglobulin is, as discussed above the whey protein gene of choice with which to "entrain" cDNA coding for the peptide of interest. In sheep, beta-lactoglobulin is the most abundantly expressed whey protein in the mammary gland and its mRNA comprises about 8% of the total polyA+ RNA. The gene has been well characterized and used to elaborate fusion contructs with cDNA sequences encoding human factor IX and human alpha1 antitrypsin.

According to a fourth aspect of the present invention, there is provided a genetic construct comprising:
(i) a DNA sequence coding for a polypeptide; and
(ii) a DNA sequence from a β-lactoglobulin gene to direct expression of the DNA sequence encoding the polypeptide in the lactating mammary gland of an adult, female, non-human mammal.

According to a fifth aspect of the present invention, there is provided a plasmid comprising a genetic construct as described above.

Preferred features of the fourth and fifth aspects are as described above for the first to third aspects, mutatis mutandis.

For the better understanding of the present invention, a number of examples will now be given. In the examples, reference will be made to the drawings in which:
FIGURES 1 to 4 show schematically the basic steps in the elaboration of a beta-lactoglobulin fusion gene in a method in accordance with the present invention;
FIGURE 5 shows a Southern blot analysis of sheep DNA;
FIGURE 5a shows a Southern blot analysis of sheep DNA and illustrates the inheritance of the beta-lactoglobulin fusion gene by the real generation.
FIGURE 6 shows an SDS-PAGE analysis of murine and ovine whey proteins; and
FIGURE 7 shows a Western blot analysis of sheep and mouse proteins.
FIGURE 8 shows a RIA of milk obtained from transgenic ewe-sheep carrying the beta-lactoglobulin fusion gene as described.

### EXAMPLE 1

### A. RECOMBINANT DNA PROCEDURES

Where not specifically detailed, recombinant DNA procedures were after Maniatis et al ("Molecular Cloning" Cold Spring Harbor (1982)) and "Methods in Enzymology" Vols 68, 100 and 101 (Wr, Grossman and Moldave, Eds.), Academic Press; and, unless specifically stated, all chemicals were purchased from BDH Chemicals Ltd, Poole, Dorset, England or the Sigma Chemical Company, Poole, Dorset, England.

### CONSTRUCTION OF OVINE BETA-LACTOGLOBULIN FUSION GENES

### Preparation of Sheep Spleen DNA

Spleen tissue was procured from a freshly slaughtered Blackface/Suffolk lamb and nuclei were isolated essentially as described by Burch and Weintraub Cell 33 65 (1983). Nuclear pellets were lysed in 0.3M NaCl, 10mM Tris.HCl, 10mM EDTA, 1% SDS pH 7.4 and 400 mcg/ml Proteinase K (Sigma Co, Fancy Road, Poole, Dorset BH17 7NH) and incubated for two hours at 37°C. Repeated phenol/chloroform extractions were performed until the preparation was completely deproteinised. The DNA was ethanol precipitated and spooled out using a glass rod, washed with 70% EtOH/30% TE (TE = 10mM Tris.HCl, 1mM EDTA pH 8.0), dried in air and resuspended in TE to a concentration of 1 mg/ml.

### Construction of Sheep Spleen DNA Lambda Fusion Genes

The lambda phage EMBL3 (Frischauf et al J. Mol. Biol. 170 827 (1983)) was employed to construct the genomic library. 30 mcg of bacteriophage DNA were digested with 5-fold excesses of the restriction enzymes EcoRI and BamHI (supplied by Amersham Intgernational plc, Lincoln Place, Green End, Aylesbury, Buckinghamshire, England) using the conditions recommended by the manufacturer. After digestion, spermine hydrochloride was added to a concentration of 5mM to precipitate the lambda DNA. After incubation for one hour on ice the DNA was pelleted at 10,000g for 15 minutes in a bench microfuge, washed in 70% EtOH, 300mM NaAc, 100mM MgCl₂, repelleted and finally resuspended in TE at a concentration of 1 mg/ml.

Sheep DNA was partially digested with the restriction enzyme Sau3A (Amersham). 100 mcg aliquots of the sheep DNA were digested with varying amounts of Sau3A [from 5-40 units] for 20 minutes at 37°C. The reactions were stopped by the addition of EDTA to 15mM. The degree of digestion was assessed by electrophoresis on 0.6% agarose gels. Suitably digested samples were pooled and loaded onto 38.0ml 10-40% sucrose gradients made up in 1M NaCl, 20mM Tris.HCl, 5mM EDTA at pH 8.0. These gradients were centrifuged in a Beckmann SW 28 rotor at 26,000 rpm for 24 hours. The sucrose gradients were fractionated from the top and 1ml fractions collected. The size distribution of DNA molecules in each fraction was assessed by agarose gel electrophoresis, and fractions containing DNA molecules from 14-21 kb in size pooled. After a two-fold dilution in TE 2 volumes of EtOH were added and the DNA precipitated overnight at -20°C. The DNA was subsequently resuspended in TE to a concentration of 300 mcg/ml.

7.5 mcg of BamHI/EcoRI cut EMBL3 and 2.5 mcg of sheep spleen DNA which had been partially digested with Sau3A were mixed together in 50 mcl of a solution containing 60mM Tris.HCl, 6mM MgCl₂, 10mM DTT, 0.01% gelatin, 0.25mM rATP and 25 units of T₄ DNA ligase (Boehringer Company, Boehringer Mannheim House, Bell Lane, Lewes, East Sussex) and incubated overnight at 14°C.

After ligation 1 mcg aliquots of the DNA were packaged in vitro using a kit purchased from Amersham following the recommended procedure of the manufacturer. The packaged library was titred on E. coli strain ED 8654. The estimated size of the library was 5.7 x 10⁶ plaque forming units (pfu's). Immediately after titration, aliquots of the unamplified library were plated onto 10 x 22cm² petri dishes (megaplates) using E. coli strain ED 8654 at a density of approximately 50,000 pfu's/plate.

### Screening the Lambda Genomic Library

Plaque-lifts from the mega-plates were performed according to the method of Benton and Davis (Science **196** 180 (1977)) onto 20cm² nitrocellulose membranes (Schleicher and Schull, Postfach 4, D-3354, West Germany). A beta-lactoglobulin cDNA clone (p931 - gift of J.C. Mercier, INRA, Jouey-en-Josas, Paris) was nick translated with ³²P dCTP to a specific activity > 10⁸ dpm/mcg, by the method described by Rigby et al (J. Mol. Biol. 113 237 (1977)). Beta-lactoglobulin cDNA may be cloned as described by Mercier et al in Biochimie 67 959-971 (1985). The sequence of the p931 clone is given by Gaye et al in Biochimie 68, 1097-1107 (1986).

Filters were prehybridised, hybridised and washed according to the method of Maniatis et al in Cell 15 687 (1978). The final wash was in 1 x SET at 68°C (SET is 0.15M NaCl, 2mM EDTA, 0.03m Tris.HCl pH 8.0). Filters were blotted dry and spotted with ³²P to orientate them before exposure to X-ray film. Regions containing positively hybridising plaques were positioned on the megaplates by reference to the ³²P spots picked using the sterile blunt end of a Pasteur pipette. The initial plaque lifts were titred on E. coli ED 8654 and plated onto 15cm diameter Petri dishes at a plaque density of approximately 500/plate. These plates were rescreened by the procedures described above and individual positively hybridising plaques were picked using a toothpick into 1.0ml of phage-buffer (phage buffer is 10mM Tris.HCl, 10mM MgCl₂, 0.01% gelatin, pH 7.4).

### Preparation of Cloned Beta-Lactoglobulin DNA

0.4ml of the resuspended phage solution was added to E. coli ED 8654 (Borg et al Mol. Gen. Genetics **146** 199-207 (1976)) and plated out on 9cm diameter Petri dishes to obtain confluent lysis of the bacterial lawn. Confluent plates were obtained from which the top plating agar was scraped off into 10ml of phage buffer and incubated overnight with a few drops of chloroform. The bacterial debris was pelleted by centrifugation at 5000 rpm for five minutes and the phage stocks stored at 4°C. The stocks were titrated on E. coli ED 8654 to determine the pfu/ml.

8 x 10⁷ pfu's were absorbed onto 7 x 10⁹ E. coli cells in 10ml of 10mM MgSO₄ at 37°C. After 15 minutes, 2.5ml aliquots were added to 100ml L Broth/10mM MgSO₄ in a one litre flask. The bacterial suspension was shaken vigorously for several hours and the OD₅₄₀ was monitored every hour. Lysis, as determined by a fall in the OD₅₄₀, occurred after several hours. When complete, 0.2ml chloroform was added to each 100ml culture and the culture left at 4°C overnight.

The bacterial debris was removed by centrifugation at 10,000 rpm for 15 minutes. 10 mcg/ml RNAase A and 10 mcg/ml DNAase I were added to the supernatant which was then incubated at 37°C for one hour. After this incubation NaCl was added to 40g/litre and polyethylene glycol (PEG) to 10%. The preparation was cooled to 4°C and left for at least two hours to precipitate the phage. The phage pellet was pelleted at 10,000 rpm for 15 minutes and resuspended in 16,0ml of phage buffer. 8.0ml of this suspension was layered upon a step gradient comprising 1.5ml 56% CsCl, 1.5ml 45% CsCl and 2.5ml 31% CsCl (dissolved in phage buffer) in a 14.0ml ultracentrifuge tube. The step gradients were centrifuged at 35,000 rpm for 1.5 hours in a swing-out rotor at 20°C. The phage band was removed with a needle and syringe and, to complete the purification of the phage particles, a second step gradient centrifugation was performed.

The purified phage particles were dialysed into 0.1M NaCl, 10mM Tris.HCl, 1mM EDTA pH 8.0 and then deproteinised by successive extractions with phenol and chloroform. NaCl was added to a final concentration of 0.3M and then the phage DNA precipitated by the addition of 2 volumes of EtOH. The DNA was pelleted by centrifugation at 10,000 rpm for 20 minutes, washed with 70% EtOH, 30% TE, dried and then resuspended in TE to a final concentration fo 200-400 mcg/ml.

### Characterisation of Recombinant Beta-Lactoglobulin Clones

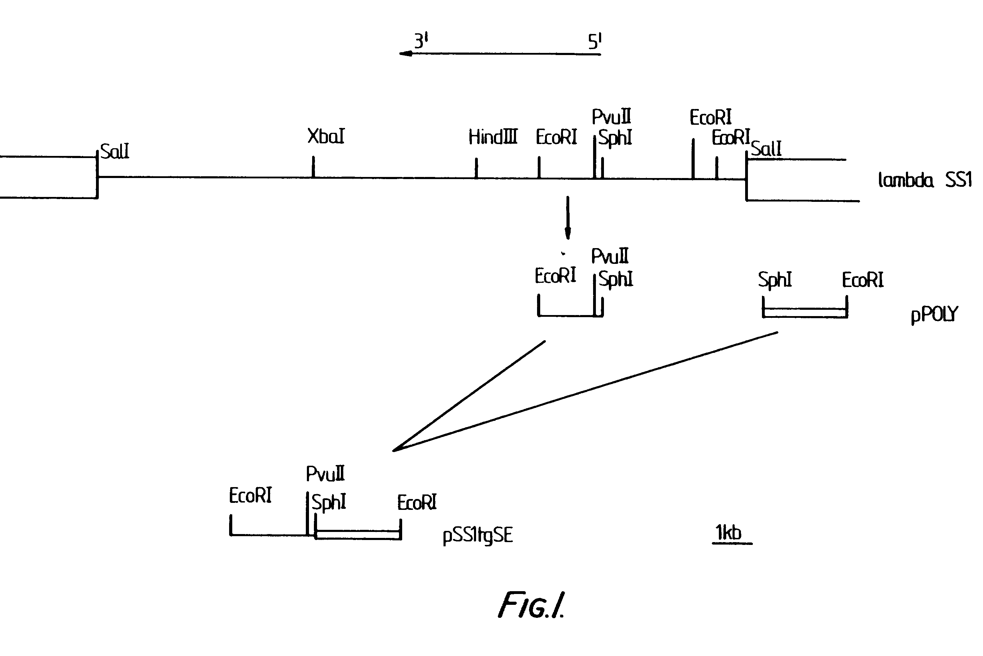

0.5 mcg aliquots of the DNA preparations described above were restricted with a variety of restriction enzymes and the products of the single and double digests analysed by electrophoresis on 0.6% and 1% agarose gels. DNA on these gels was transferred to nitrocellulose filters on to Hybond membranes (Amersham International, Little Chalfont, Bucks) by the method fo Southern (J. Mol. Biol. 98 503 (1975)) and hybridised to ³²P labelled p931. The procedure used was essentially as described above and the hybridised filters were analysed by autoradiography. Using a variety of restriction enzymes and specific probes from the 5' and 3' ends of p931 a restriction map was constructed in which the size and orientation of the beta-lactoglobulin gene(s) was determined, (see Figure 1).

The identity of the beta-lactoglobulin clones and the precise position of the 5' and 3' ends of the gene were directly confirmed by DNA sequencing. Using suitable restriction sites, fragments were subcloned into plasmid vectors and into M13 vectors. Sequencing was carried out using the dideoxy method of Sanger et al. (PNAS **74** 5463 (1977)).

### Elaboration of Beta-Lactoglobulin Fusion Genes

The strategy used for elaborating fusion genes comprising beta-lactoglobulin and genes of interest to be expressed in the mammary gland is outlined in Figures 1 to 4. The approach utilises sequences derived from a lambda clone, whose isolation and characterisation is described above. The strategy involves insertion of the DNA sequence of interest into the region of DNA corresponding to the 5' untranslated region of beta- lactoglobulin mRNA. Proteins translated from mRNA transcripts of this gene will contain the secretory peptide of the target protein.

The subclone pSS-1tg SE was constructed as shown in Figure 1 by ligating the 1.4 kb SphI-EcoRI fragment of lambda phage SS-1 into the vector plasmid pPoly which had also been cut with SphI + EcoRI and E. coli strain DH1 transformed having been made competent by the procedure of Hanahan and Meselson (Gene **10** 63 (1980)). Ampicillin resistant clones were isolated and DNA prepared from them by the method of Birnboim and Doty (Nuc. Acid Res. **7** 1513 (1979)).

In Figure 1, the top arrow defines the orientation and extent (approximately 4.9 kb) of the beta-lactoglobulin transcription unit present within lambda SS1; the scale is the same throughout. More generally, in Figures 1 to 4, it should be noted that only relevant restriction sites are shown. Large open boxes represent lambda EMBL3 arms; narrow open boxes represent pPoly; the narrow shaded box represents the target sequence to be expressed; lines represent cloned sheep sequences corresponding to the beta-lactoglobulin gene and its flanking sequences.

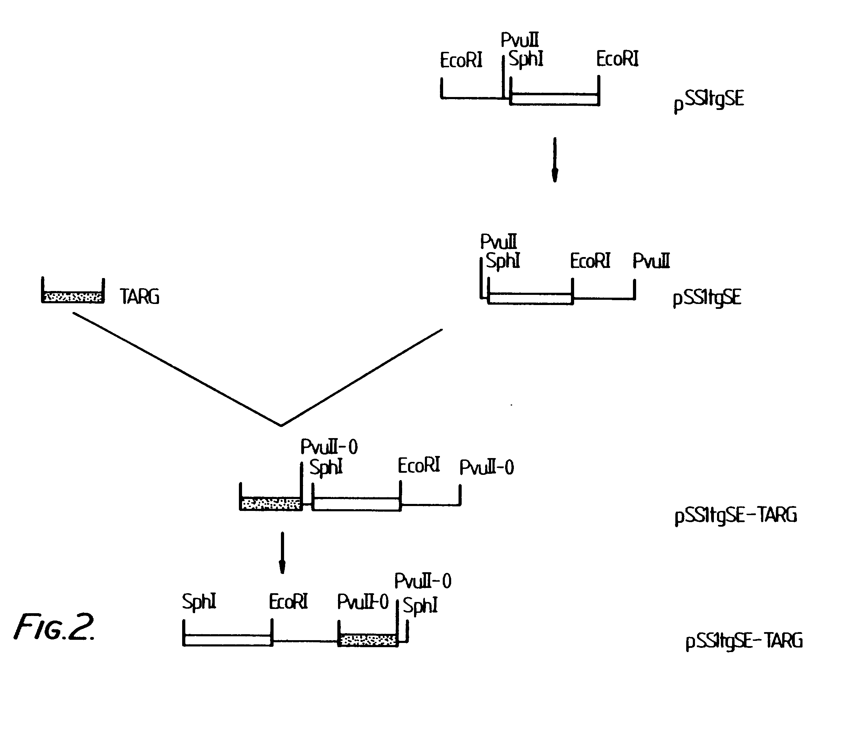

pSS-1tgSE was linearised by digestion with the restriction endonuclease PvuII (Figure 2) which cuts at a unique site in the plasmid in a region of DNA corresponding to the 5' untranslated mRNA sequences of beta-lactoglobulin. 5 mcg of completely digested plasmid was dissolved in 0.5M Tris.HCl, 10mM MgCl₂, 1mM MnCl₂, 10mM spermidine, pH 9.0, and treated with 0.01-0.04 units of calf intestinal phosphatase (Boehringer) for 30 minutes at 56°C. The calf intestinal phosphatase was inactivated in 0.5% SDS and the DNA recovered by phenol/chloroform extractions and EtOH precipitation.

A Factor IX DNA clone p5'G3'CVI was procured from Dr. G. Brownlee, Sir William Dunn School of Pathology, University of Oxford, Oxford. This clone contains a 1579 bp insert in a plasmid derived from pAT153 (Twig et al Nature **283**, 216-218 (1980)). It runs from the TagI site at -7 from the presumed mRNA start site to +1572 and contains the entire coding sequence for human Factor IX (Anson et al Embo J. 3, 1053-1060 (1984)).

A NheI-HindIII fragment, comprising 1553 bp of Factor IX sequences was excised and purified from the vector sequences by the methods described below. The HindIII and NheI ends of this were blunted using Klenow polymerase by the technique described by Maniatis et al ("Molecular Cloning" Cold Spring Harbor (1982)) and the fragment ligated into PvuII restricted, phosphatased pSS1tgSE (described above) to form pSS1tgSE-Factor IX after transforming E. coli DH-1 to ampicillin resistance.

The Factor IX cDNA sequence is hereafter designated TARG, as can be seen in Figure 2.

Plasmid DNA was prepared as described above and checked by digestion with suitable restriction enzymes. The plasmid DNA was digested with SphI + EcoRI, electrophoresed on a 1% agarose gel containing 0.5 mcg/ml ethidium bromide (Sigma). The relevant SphI-EcoRI fragment was located by illumination with a UV lamp (Ultra-Violet Products, Inc., San Gabriel, California, USA). A piece of dialysis membrane was inserted in front of the band and the DNA subsequently electrophoresed onto the membrane. The DNA was eluted from the dialysis membrane and isolated by use of an "Elu-tip" [Schleicher and Schull, Postfach 4, D-3354, Dassel, W. Germany], employing the procedure recommended by the manufacturer.

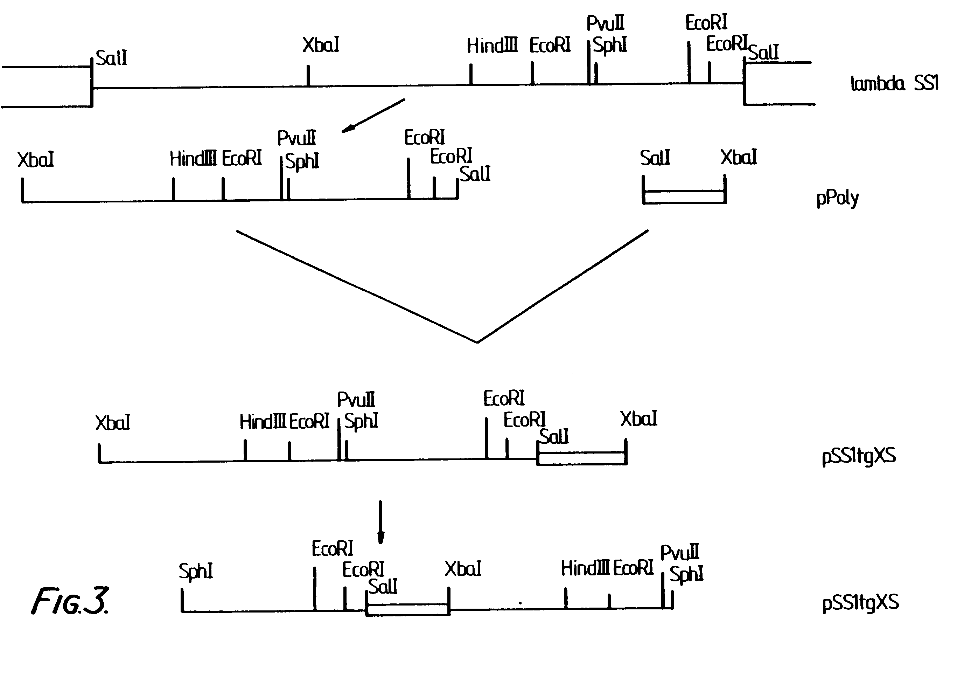

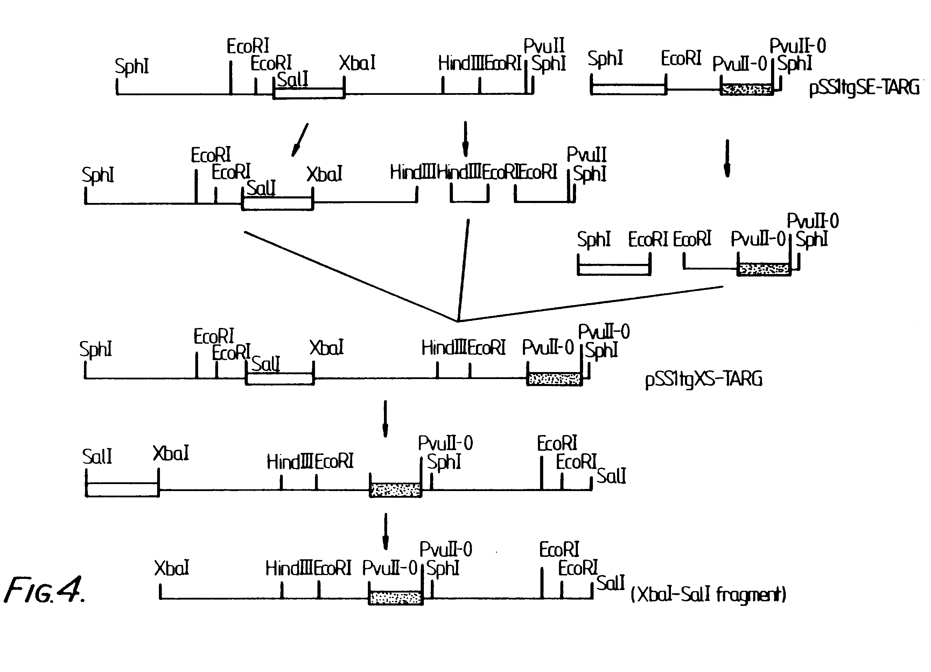

The plasmid pSSItgXS (Figure 3) was constructed by ligating the XbaI-SalI fragment of lambda SS-1 into XbaI-SalI digested pPoly. Clones were isolated and plasmid DNA prepared as previously described. Two DNA fragments were independently isolated from this plasmid or smaller subclones derived from it: a 10.5 kb SphI (partial) HindIII fragment and a 1.2 kb EcoRI-HindIII fragment (Figure 4). These fragments were isolated by gel electrophoresis, as described above.

The SphI-EcoRI, SphI-HindIII, and the EcoRI-HindIII fragments were ligated together in approximately equal ratios and the DNA used to transform DH-1. Plasmid DNA (pSSItgXS-TARG) was prepared as described above and digested with xbaI and SalI to excise the beta-lactoglobulin fusion gene from the vector. This fragment was purified by gel electrophoresis followed by the use of an "Elu-tip". The ethanol precipitated DNA was pelleted, resuspended in TE and phenol/chloroform extracted and reprecipitated. The DNA was finally resuspended in TE and was employed directly for microinjection.

B. CONSTRUCTION OF TRANSGENIC ANIMALS

Collection of Fertilised Eggs

Procedures were analogous to those described for mice in Gordon and Ruddle in "Methods in Enzymology" (1983) Vol 101 (W, Grossman and Moldave, Eds.) Academic Press pp411 to pp432.

Embryos are recovered from ewes that have been induced to superovulate at an oestrus that is controlled by treatment with progestagen. Mature ewes of proven fertility are treated for 12-16 days with intravaginal sponges that are impregnated with 60mg medrogproxy-esterone acetate (Veromix, Upjohn Ltd. Crawley). Equine follicle stimulating hormone [3.5-4.3mg in aqueous solution/ewe] is given in 2 equal intramuscular injections 28 hrs before the end of progrestagen treatment and at the time of sponge withdrawal. Ewes are allowed to mate several times at the oestrus that occurs 20-72 hrs after sponge removal. The ewes are observed for the onset of heat at 08.00, 12.00, 16.00 and 20.00 hr daily. Embryos at the 1 to 4 cell stages of development are recovered during surgery 36-72 hrs after the onset of oestrus.

Anaesthesia is induced by intravenous injection of thiopentone sodium [Intraval, May and Baker] and maintained by mixtures of oxygen and nitrous oxide in a semi-closed circuit system. Embryo recovery was carried out by the procedures of Hunter et al (J. Agric. Sci. **46** 143-149 (1955)). The reproductive tract is exposed through a mid-central incision and a nylon catheter inserted into the oviduct through the fimbria. Medium is introduced into the uterine lumen through a blunted 18-gauge needle and forced through the uterotubal junction and along the oviduct. The embryos are recovered in phosphate-buffered saline containing energy sources and protein [Ovum Culture Medium, Flow Labs, Irvine, Scotland]. During storage and microinjection of eggs this medium is supplemented with 20% foetal calf serum.

### Injection of DNA

DNA (1 to 2 mcg/ml) is injected into one pronucleus of single cell eggs or into one or more nuclei of 2 and 4 cell eggs. The eggs are manipulated in a chamber filled with ovum culture medium. The chamber consists of a siliconised microscope slide with glass supports (25mm x 2mm x 3mm) parallel to the long side of the slide. A coverslip is mounted on top of the supports, the junctions being sealed with silicone grease. The open ends of the chamber are filled with Dow-Corning 200 fluid (50cs) (BDH Chemicals).

Eggs to be injected are held by suction on a blunt glass pipette. Pronuclei or nuclei are visualised using a Nikon Diaphet inverted microscope (Nikon (UK) Ltd, Haybrooke, Halesfield 9, Telford, Shropshire). DNA is injected into pronuclei or nuclei using a micropipette drawn from capillary tubing (borosilicate glass - 1mm external diameter, thin wall with filament - Clark Electromedical Instruments, PO Box 8, Pangbourne, Reading, RG8 7HU) on a microelectrode puller (Campden Instruments, 186 Campden Hill Road, London). The positions of the two microinstruments are controlled using micro-manipulators (Leitz Mechanical Micromanipulators, E. Leitz (Instruments) Ltd, 48 Park Street, Luton, England). The micropipette containing the DNA to be injected is connected via air-tight tubing to a 100 ml glass syringe. Injection is performed by application of pressure using the syringe. Successful injection is indicated by visible swelling of the pronuclei or nuclei. The injected eggs are incubated at room temperature for a minimum of 30 minutes to allow visible degeneration of damaged eggs.

Embryos judged to have survived injection are transferred to un-mated recipient ewes whose oestrus cycles are synchronised with those of egg donors by treatment with progestagen [Veromix, Upjohn Ltd]. Embryos are transferred to the oviduct using fine-drawn mouth-pipettes. Up to 4 embryos are transferred to each ewe. The embryos are distributed between the oviducts.

The body-wall of the ewes is closed with soluble filament [Descon, Davis and Greck] and the skin with Michel clips. Each ewe is given antibiotics [Duphapen L.A., Duphar, Amersham] at the time of surgery. The Michel clips are removed 10-20 days after surgery.

### Development and Growth

After recovery from anaesthesia the ewes are returned to the paddock where they remain throughout pregnancy. Supplementary hay, turnips and concentrates are provided as required. During the third month of pregnancy the number of fetuses is determined by real ultra-sonic scanning. (White et al Vet. Rec. 115, 140-143 (1986)). Management of pregnant ewes is then adjusted to take account of variation in foetal number. As the expected date of parturition approaches the ewes are housed to facilitate supervision and assistance during lambing.

### Analysis of Transgenic Lambs

At least 2 weeks after birth 10 ml samples of blood are removed by venous puncture with a hypodermic syringe and collected in a heparinised tube. DNA was prepared from blood samples as follows:- 30 ml of lysis solution (155mM NH₄Cl, 10mM KHCO₃, 1 mM EDTA) was added to a 10 ml blood sample and the mixture incubated for 15 minutes on ice. The white blood cells were spun down at 1500g for 10 minutes at 40°C, resuspended in 10ml SE (75mM NaCl, 2mM EDTA) and then washed once in SE. Proteinase K was added to 100 mcg/ml followed by 1ml 20% SDS and the preparation incubated for 4 hours. Repeated phenol/chloroform extractions were performed until the preparation was completely deproteinised. 1/30th vol of 0.3M NaAc-1 vol isopropanol were added to the aqueous phase to precipitate the DNA, which was hooked out, rinsed in 70% EtoH and resuspended in TE.

10mcg aliquots of the DNA preparations were digested with suitable restriction enzymes (eg EcoRI) and electrophoresed on 0.8% gels. These gels were analysed by Southern Blotting and hybridisation was performed essentially as described.

Positively hybridising animals, ie those containing the fusion gene (presumed to be integrated at a chromosomal location), are allowed to grow to maturity. Females are mated and, once lactating, their milk analysed for the substance of interest (see Example 7). Positive males are mated and their daughters screened for the exogenous DNA sequences and their milk is subsequently analysed for the substance of interest.

EXAMPLE 2

The procedure of Example 1 is repeated, except that the DNA sequence encoding the polypeptide of interest (the TARG sequence) encodes alpha₁-antitrypsin was obtained from R. Cortese, EMBL, Meyerhofstrasse 1, D-6900 Heidelberg, West Germany. A TagI-BstNI fragment comprising 1294 bp of the insert (see Ciliberto et al Cell **41** 531-540 (1985)) was excised, purified from this clone and cloned into the PvuII site of pSSItgSE by the methods described in Example **1**.

### EXAMPLE 3

The procedure of Example 1 is repeated, except that instead of the plasmid pPoly, the plasmid pUC18 (Pharmacia Ltd, Pharmacia House, Midsummer Boulevard, Milton Keynes", England) is used instead.

### EXAMPLE 4

The procedure of Example 1 is repeated, except that instead of the plasmid pPoly, the plasmid pUC19 (Pharmacia Ltd, Pharmacia House, Midsummer Boulevard, Milton Keynes", England) is used instead.

### EXAMPLE 5

### Generation of Transgenic Sheep

The SalI-XbaI fragment, excised from the plasmid pSS1tgXS-FIX (also designated pSS1tgXS-TARG, see Example 1), was injected into sheep eggs. Approximately 200 copies/fertilised egg were injected. From 252 one cell eggs injected and reimplanted into recipient ewes, 52 live lambs were born. Based on the analysis of DNA prepared from blood samples four of these animal were shown to carry the exogenous betalactoglobulin- Factor IX sequences (Table 4).

**TABLE 4**

| Summary of Transgenic Sheep. | | | |
|---|---|---|---|
| LAMB No. | SEX | CONSTRUCT | COPY No. (Approx) |
| 6LL225 | M | BLG-FIX | 40 |
| 6LL231 | F | BLG-FIX | 10 |
| 6LL239 | M | BLG-FIX | 1 |
| 6LL240 | F | BLG-FIX | 10 |

All of the eggs were injected at the pronucleus stage and none were centrifuged. Copy numbers were determined by quantitative scanning densitometry.

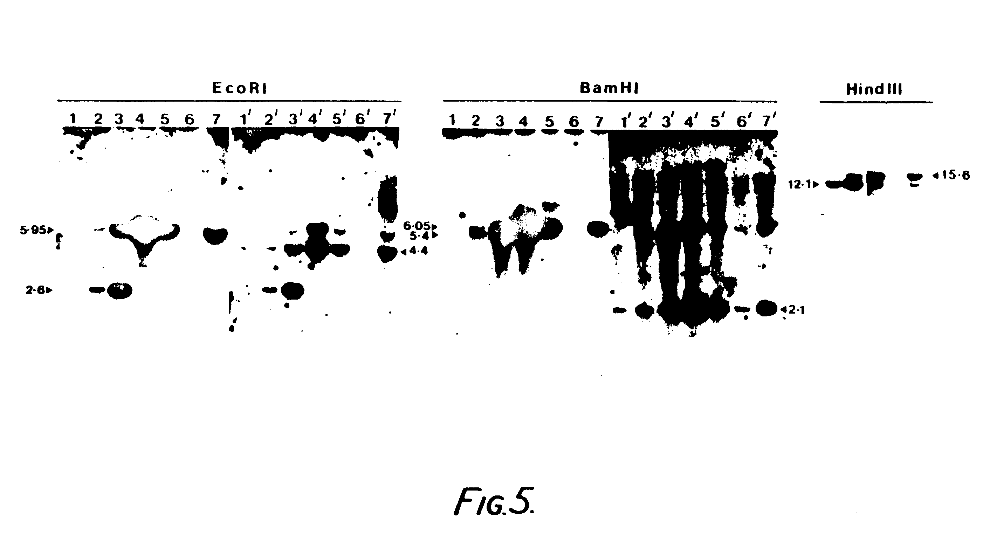

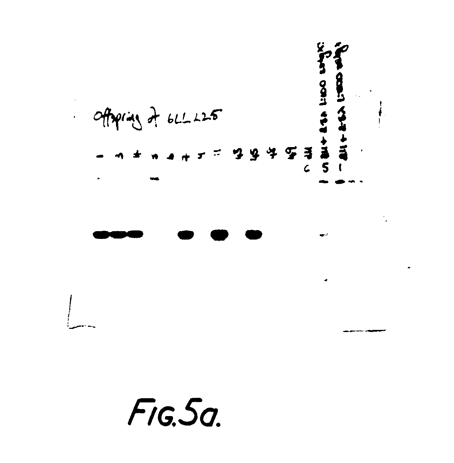

Figure 5 is a Southern blot analysis of the sheep DNA. Sheep DNA was prepared as described, digested with the restriction enzymes indicated, electrophoresed on a 0.8% agarose gel and transferred to Hybond membrane (Amersham International, Little Chalfont, Bucks, UK). The filter was probed with 32P labelled plasmid pSS1tgXS-FIX (lanes 1 - 7) and subsequently (after stripping the membranes) with plasmid p931 (lanes 1' - 7'). Copy controls of pSS1tgXS-FIX were included on the gel where indicated. Lane 1, control (nontransgenic) sheep DNA; lanes 2 and 3 control DNA plus 1 and 5 copy equivalents of p5'G3'CVI; lanes 4-7, DNA from transgenic sheep 6LL225, 6LL231, 6LL239 and 6LL240. Each transgenic sheep yields Factor IX hybridising bands of 5.95 kb (EcoRI) and 6.05 kb (BamHI), identical in size to those derived from pSS1tgXS-FIX, showing that the 5' ends of the transgenes are intact. Significant hybridisation with sheep Factor IX was not observed. Hybridisation with p931 revealed the predicted 4.4 kb EcoRI and 2.1 kb BamHI fragments. Although the endogenous sheep betalactoglobulin genes contribute to the hybridisation in these bands, the increased intensity in samples from 6LL225, 6LL231 and 6LL240 indicate that these bands derive primarily from the injected betalactoglobulin-Factor IX fusion gene, confirming the integrity of the 3' ends of the construct. HindIII cleaved DNA: lane 1, 12.1 kb purified SalI-XbaI fragment excised from pSS1tgXS-FIX; lanes 2-5 DNA from 6LL225, 6LL231, 6LL239 and 6LL240. The probe was p5'G3'CVI. The hybridising 12.1 kb HindIII fragment (6LL225, 6LL231 and 6LL240), identical in size with the injected fragment indicates a head to tail arrangement; the 15.6 kb fragment common to the same sheep indicates that head to head repeats are also present. These data indicate that in sheep 6LL225, 6LL231 and 6LL240 the SalI-XbaI fragment derived from pSS1tgXS-FIX (the betalactoglobulin-Factor IX fusion gene) has integrated without detectable rearrangement in tandem arrays. In sheep 6LL239 the data is compatible with the integration of a single unrearranged copy of this fragment.

### EXAMPLE 6

### Transmission of Factor IX Sequences to the Next Generation

The male transgenic sheep 6LL225 from Example 5 (carrying approximately 40 copies of the SalI-XbaI fragment prepared from pSS1tgXS-FIX) was successfully mated to a number of Finn-Dorset and East Friesland ewes. His progeny were analysed by Southern blotting of DNA prepared from blood samples using the human Factor IX plasmid probe p5'G3'CVI, as described. A Southern blot of this analysis on some of his progeny is shown in Figure 5a. Lanes numbered 1,3,4,5,6,7,9,11,43,45,48 and 49, DNA from progeny of 6LL225 (designated 7R1, 7R3 etc - 7R49), lane c 211, DNA from control (nontransgenic) sheep 6LL211, lanes 5-211 and 1-211, DNA from sheep 6LL211 plus 5 and 1 copy equivalents of pSS1tgXS-FIX. These data show that transgenic sheep 6LL225 has transmitted the Factor IX sequences to 6 out of 12 of his progeny and, therefore that these sequences have been incorporated into the germline.

### EXAMPLE 7

### Expression of the Gene Encoding Ovine Beta Lactoglobulin in Transgenic Mice

Transgenic mice were generated essentially by the techniques described in Gordon and Ruddle, in Methods in Enzymology Vol 101 (1983), (Eds. Wu, Grossman and Moldave), Academic Press pp411-432. Several transgenic mice carrying the SalI fragment of the clone lambda SS-1 (Fig. 3) were produced. One of these, B-Lac 7, a female was shown to carry 15-20 copies of the SalI fragment. B-lac 7 was mated a number of times and produced a number of offspring which inherited the SS-I sequences.

Milk was obtained from mice 8-12 days after the birth of a litter. This was accomplished by intra-peritoneal injection of 0.3IU oxytocin (Sigma) & 7 mcl/g animal of Hypnorm/Hypnovel (Fleckneil, Vet. Rec. Dec 10 1983, p574), after having previously removed the pups for a four hour period, waiting 20 minutes and then massaging the individual mammary gland by hand. Milk was collected in a 50 mcl capillary tube.

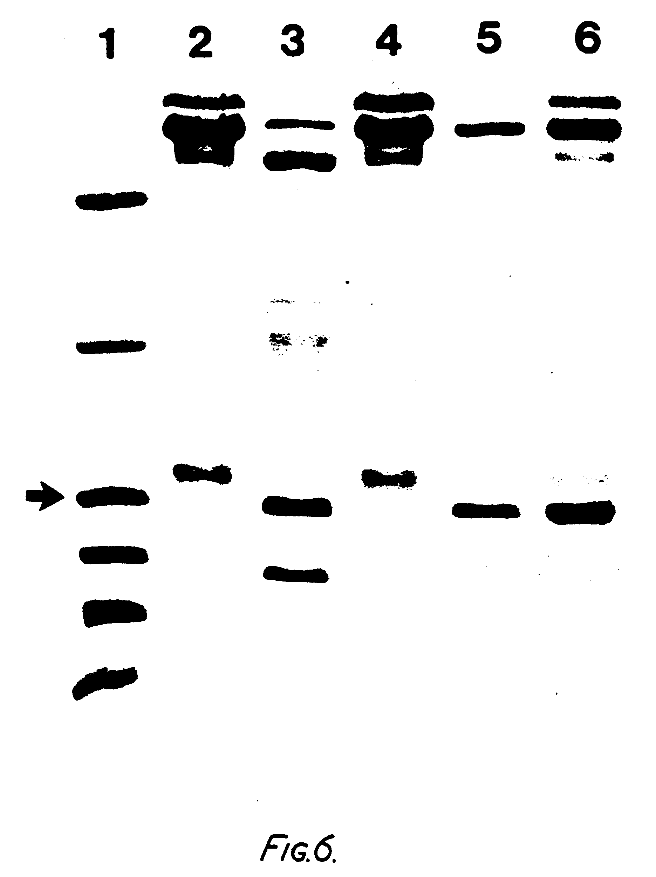

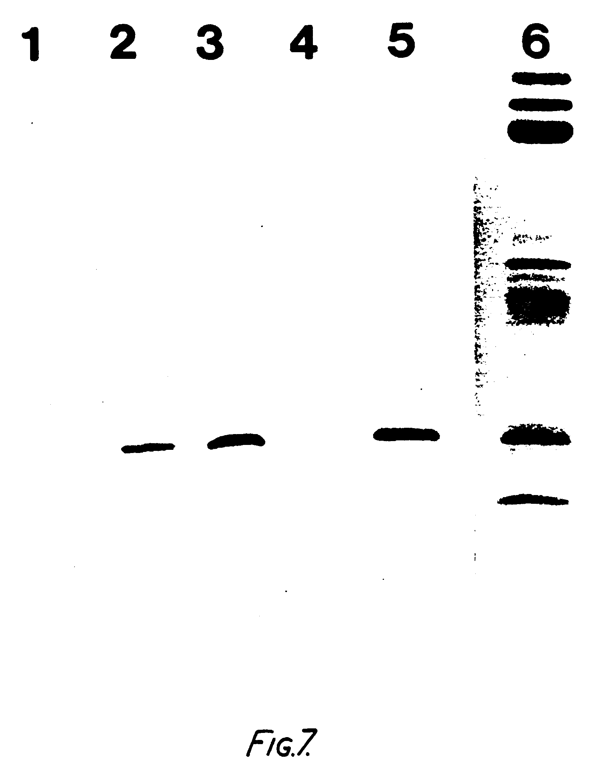

The mouse milk was diluted 1:5 in distilled water, centrifuged briefly in a bench centrifuge to defat and the caseins precipitated by addition of 1N HCl to a final pH of 4.6. After centrifugation in a bench centrifuge the whey proteins were removed, precipitated with 5% trichloracetic acid and analysed by polyacrylamide gel electrophoresis according to Laemmli (Nature **277**, 680-684 (1970). (Figure 6 shows an SDS PAGE Analysis of Murine and Ovine Whey Proteins. Lane 1, marker proteins; 2, normal mouse whey; 3, sheep whey; 4, normal mouse whey; 5, B-lac7 whey; 6, B-lac7 whey (2.5 x 5). The band corresponding to beta lactoglobulin in the marker track and in ovine whey is arrowed.) Anti-sera raised in rabbits against ovine beta-lactoglobulin was used to detect ovine beta-¶ lactoglobulin by Western blotting (Burnett, Anal. Bioches., 112, 195-203, (1981)) on samples resolved by gel electrophoresis. Figure 7 shows a Western Blot Analysis. The Western blot was reacted with rabbit anti-beta-lactoglobulin serum and anti-rabbit Ig peroxidase serum. (Lane 1, marker proteins; 2, sheep whey; 3, B-lac7 whey; 4, normal mouse whey; 5, purified beta-lactoglobulin; 6, Coomassie stained sheep whey (run in parallel)).

This analysis showed that large amounts of beta-lactoglobulin were secreted into mouse milk, indicating that SS-1 was being expressed at high levels in B-lac 7. This clone presumably contains all the necessary sequences to ensure high levels of expression in the mammary gland of a transgenic mouse and can thus be expected to function as efficiently, if not more so, in the homologous species ie in a transgenic sheep. Consequently, fusion genes derived from this clone can also be expected to express (efficiently) in the ovine mammary gland.

### EXAMPLE 8

### Expression of Human Factor IX in the Milk of Transgenic Ewes

Two female sheep, 6LL231 and 6LL240 (each carrying approximately 10 copies of SalI-XbaI fragment prepared from pSS1tgXS-FIX) were successfully mated to East Friesland rams. After birth the lambs were allowed to suckle naturally for about two weeks to stimulate lactation. Milk (approximately 25 ml from each animal) was collected by hand into sterile plastic containers. Milk from a control (nontransgenic) lactating ewe was also collected. The samples were frozen at -20'C and delivered to the Scottish National Blood Transfusion Service at the Royal Infirmary, Edinburgh, where radioimmunoassays (RIA's) for human Factor IX were performed.

Transgenic and control milk were dialysed against distilled water overnight at 4°C and then freeze-dried. Freeze-dried sample were resuspended in distilled water and then centrifuged to separate the cream and examined by RIA for the presence of human Factor IX as follows:-

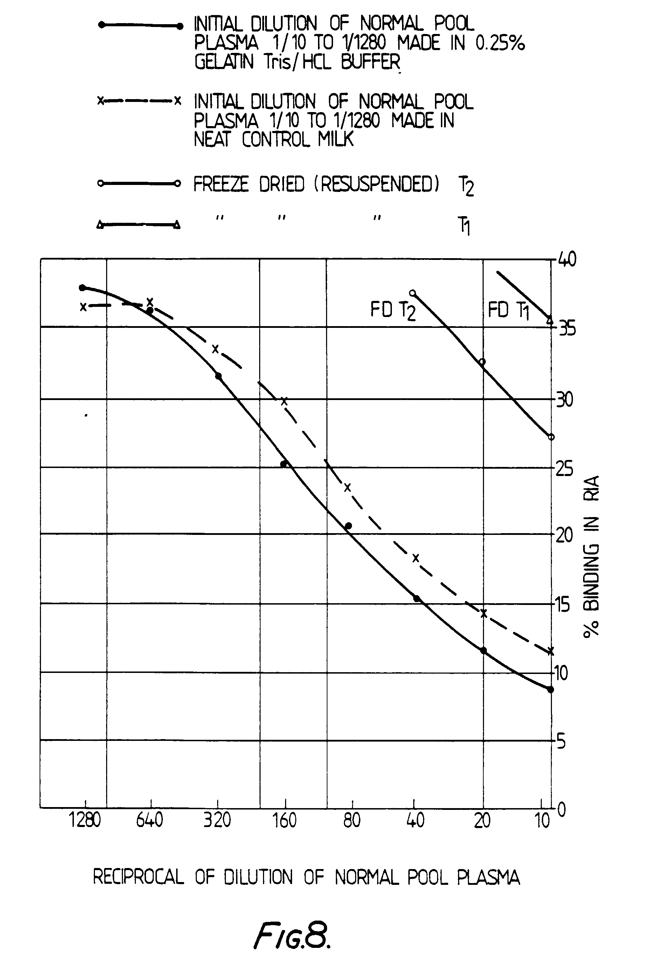

A standard curve, using normal human pooled plasma diluted in RIA buffer (50mM Tris/HCl, 0.25% gelatin, 1% Tween-20, 10mM HCl, pH 7.2) was established using dilutions of 1/10 - 1/1280 (see Figure 8). In order to rule out any interference by milk in the assay, normal pool plasma was similarly diluted in control milk, and a standard curve established. Dilutions of samples of freeze-dried milk from 6LL231 and 6LL240 were assayed. Each tube in the RIA comprised the following:- 50 mcl RIA buffer, 50 mcl sample dilution, 50 mcl rabbit polyclonal anti-Factor IX antibody (Dako-Patts), at a 1/30000 dilution and 50 mcl ¹¹²⁵ labelled Factor IX. A control for maximum binding was set up comprising 100 mcl RIA buffer, 50 mcl antibody and 50 mcl of 1125 Factor IX. A control for non-specific binding was also set up comprising 150 mcl RIA buffer, 50 mcl 1125 trace.

After overnight incubation 50 mcl of Sepharose-S1000 conjugated with donkey anti-rabbit IgG was mixed in the tubes and the beads recovered by sucrose separation. The RIA assay is sensitive to about 0.125 international units (iu)/dl. Any sample containing Factor IX above this level will inhibit maximum binding in the RIA.

The % binding in the RIA was plotted against the reciprocal of the dilution factor for the following samples:-
1. Normal pool plasma
2. Normal pool plasma plus milk
3. Freeze dried milk samples from 6LL231 (T1)
4. Freeze dried milk samples from 6LL240 (T2)

These results are shown in Figure 8.

Milk from both 6LL231 (T1) and 6LL240 (T2) exhibited detectable levels of Factor IX at 2.5 iu/dl and 8.0 iu/dl, respectively. No activity was detected in control milk at the level of sensitivity of the assay. These data show that transgenic ewes carrying the betalactoglobulin-factor IX fusion gene (specifically the SalI-XbaI fragment derived from pSS1tgXS-FIX (also generally designated pSS1tgXS-TARG)) express this gene and secrete the human protein into the milk. This establishes a basis for the production of human proteins in this manner.

## Claims (Claims for the following Contracting State(s): BE, CH, LI, DE, FR, GB, IT, LU, NL, SE)

1. A method of producing in milk a substance comprising a polypeptide, the method comprising:
(a) producing milk in the mammary gland of an adult, female, non-human mammal whose genetic material includes a genetic construct comprising:
(i) a DNA sequence coding for the polypeptide; and
(ii) a DNA sequence from a β-lactoglobulin gene to direct expression of the DNA sequence coding for the polypeptide; and
(b) collecting the milk produced in step (a).

2. A method as claimed in claim 1, wherein the substance is subsequently recovered from milk of the adult female mammal.

3. A method as claimed in claim 2, wherein the substance is recovered from the milk after post-translational modification.

4. A method as claimed in claim 1, 2 or 3 wherein the substance comprising a polypeptide is a protein.

5. A method as claimed in claim 4, wherein the substance comprising a polypeptide is a human blood protein.

6. A method as claimed in claim 4, wherein the substance comprising a polypeptide is a peptide hormone.

7. A method as claimed in claim 5, wherein the substance comprising a polypeptide is a blood coagulation factor or a subunit of a blood coagulation factor.

8. A method as claimed in any one of claims 1 to 4, wherein the substance comprising a polypeptide is an enzyme.

9. A method of producing in milk a substance which is the reaction product of an enzyme, the method comprising:
(a) producing milk in the mammary gland of an adult, female, non-human mammal whose genetic material includes a genetic construct comprising:
(i) a DNA sequence coding for the enzyme; and
(ii) a DNA sequence from a milk whey protein gene to direct expression of the DNA sequence coding for the enzyme; and
(b) allowing the enzyme to catalyse the formation of the reaction product from one or more substrates of the enzyme.

10. A method as claimed in claim 9, wherein the milk whey protein gene is a β-lactoglobulin gene.

11. A method as claimed in claim 9 or 10, wherein the reaction product is recovered from milk of the adult female mammal.

12. A process for the production of a transgenic mammal, the process comprising:
(a) incorporating *in vitro* in a genetic construct comprising:
(i) a DNA sequence coding for a polypeptide; and
(ii) a DNA sequence from a β-lactoglobulin gene to direct expression of the DNA sequence coding for the polypeptide in the mammary gland of an adult, female mammal;
(b) incorporating the genetic construct into a non-human mammal's germline by injection into a fertilised egg or a cell of an embryo of the non-human mammal; and
(c) allowing the injected fertilised egg or embryo to develop into an adult mammal; and
(d) optionally breeding a female mammal, whose germline contains the genetic construct from the mammal produced in step (c).

13. A process as claimed in claim 12, wherein the injection is made into a nucleus of a two-cell embryo.

14. A process as claimed in claim 12, wherein linear molecules of DNA are injected into a pro-nucleus or a nucleus of the egg.

15. A method as claimed in any one of claims 1 to 11, or a process as claimed in claims 12, 13 or 14, wherein the mammal is a domestic livestock mammal.

16. A method or process as claimed in claim 15, wherein the mammal is selected from members of the family *Suidae,* members of the genus *Ovis,* members of the genus *Capra,* and members of the genus *Bos.*

17. A method or process as claimed in claim 15, wherein the mammal is a dairy sheep.

18. A method or process as claimed in any one of claims 1 to 17, wherein the β-lactoglobulin is ovine β-lactoglobulin.

19. A method or process as claimed in any one of claims 1 to 18, wherein the genetic construct comprises a promoter.

20. A method or process as claimed in any one of claims 1 to 19, wherein the genetic construct comprises a start site for transcription.

21. A method or process as claimed in any one of claims 1 to 20, wherein the genetic construct comprises one or more distal 5' regulatory sequences of the milk whey protein gene.

22. A method or process as claimed in any one of claims 1 to 21, wherein the genetic construct comprises a structural milk whey protein gene sequence optionally including internal regulatory sequences.

23. A method or process as claimed in any one of claims 1 to 22, wherein the genetic construct comprises a 3' sequence flanking the milk whey protein gene.

24. A method or process as claimed in any one of claims 1 to 23, wherein the genetic construct comprises a cDNA sequence coding for the polypeptide of interest inserted into the first exon of the gene encoding the milk whey protein.

25. A method or process as claimed in any one of claims 1 to 24, wherein the genetic construct includes some of the 5' flanking sequences of the gene encoding the milk whey protein.

26. A method or process as claimed in any one of claims 1 to 25, wherein the genetic construct contains a sequence encoding a signal peptide for the polypeptide of interest.

27. A method or process as claimed in claim 26, wherein the sequence encoding a signal peptide of the milk whey protein gene is precisely fused to that portion of the DNA sequence that encodes the N-terminal amino acid of the mature polypeptide in the substance produced.

28. A method or process as claimed in any one of claims 1 to 27, wherein the 3' end of the DNA sequence terminates after its stop codon, but before its own polyA addition site.

29. A genetic construct comprising:
(i) a DNA sequence coding for a polypeptide; and
(ii) a DNA sequence from a β-lactoglobulin gene to direct expression of the DNA sequence encoding the polypeptide in the lactating mammary gland of an adult, female, non-human mammal.

30. A genetic construct as claimed in claim 29, comprising any or all of the specific features of claims 19 to 28.

31. A plasmid comprising a genetic construct as claimed in claim 29 or 30.

## Claims (Claims for the following Contracting State(s): AT)

1. A method of producing in milk a substance comprising a polypeptide, the method comprising:
(a) producing milk in the mammary gland of an adult, female, non-human mammal whose genetic material includes a genetic construct comprising:
(i) a DNA sequence coding for the polypeptide; and
(ii) a DNA sequence from a β-lactoglobulin gene to direct expression of the DNA sequence coding for the polypeptide; and
(b) collecting the milk produced in step (a).

2. A method as claimed in claim 1, wherein the substance is subsequently recovered from milk of the adult female mammal.

3. A method as claimed in claim 2, wherein the substance is recovered from the milk after post-translational modification.

4. A method as claimed in claim 1, 2 or 3 wherein the substance comprising a polypeptide is a protein.

5. A method as claimed in claim 4, wherein the substance comprising a polypeptide is a human blood protein.

6. A method as claimed in claim 4, wherein the substance comprising a polypeptide is a peptide hormone.

7. A method as claimed in claim 5, wherein the substance comprising a polypeptide is a blood coagulation factor or a subunit of a blood coagulation factor.

8. A method as claimed in any one of claims 1 to 4, wherein the substance comprising a polypeptide is an enzyme.

9. A method of producing in milk a substance which is the reaction product of an enzyme, the method comprising:
(a) producing milk in the mammary gland of an adult, female, non-human mammal whose genetic material includes a genetic construct comprising:
(i) a DNA sequence coding for the enzyme; and
(ii) a DNA sequence from a milk whey protein gene to direct expression of the DNA sequence coding for the enzyme; and
(b) allowing the enzyme to catalyse the formation of the reaction product from one or more substrates of the enzyme.

10. A method as claimed in claim 9, wherein the milk whey protein gene is a β-lactoglobulin gene.

11. A method as claimed in claim 9 or 10, wherein the reaction product is recovered from milk of the adult female mammal.

12. A process for the production of a transgenic mammal, the process comprising:
(a) incorporating *in vitro* in a genetic construct comprising:
(i) a DNA sequence coding for a polypeptide; and
(ii) a DNA sequence from a β-lactoglobulin gene to direct expression of the DNA sequence coding for the polypeptide in the mammary gland of an adult, female mammal;
(b) incorporating the genetic construct into a non-human mammal's germline by injection into a fertilised egg or a cell of an embryo of the non-human mammal; and
(c)- allowing the injected fertilised egg or embryo to develop into an adult mammal; and
(d) optionally breeding a female mammal, whose germline contains the genetic construct from the mammal produced in step (c).

13. A process as claimed in claim 12, wherein the injection is made into a nucleus of a two-cell embryo.

14. A process as claimed in claim 12, wherein linear molecules of DNA are injected into a pro-nucleus or a nucleus of the egg.

15. A method as claimed in any one of claims 1 to 11, or a process as claimed in claims 12, 13 or 14, wherein the mammal is a domestic livestock mammal.

16. A method or process as claimed in claim 15, wherein the mammal is selected from members of the family *Suidae,* members of the genus *Ovis,* members of the genus *Capra,* and members of the genus *Bos.*

17. A method or process as claimed in claim 15, wherein the mammal is a dairy sheep.

18. A method or process as claimed in any one of claims 1 to 17, wherein the β-lactoglobulin is ovine β-lactoglobulin.

19. A method or process as claimed in any one of claims 1 to 18, wherein the genetic construct comprises a promoter.

20. A method or process as claimed in any one of claims 1 to 19, wherein the genetic construct comprises a start site for transcription.

21. A method or process as claimed in any one of claims 1 to 20, wherein the genetic construct comprises one or more distal 5' regulatory sequences of the milk whey protein gene.

22. A method or process as claimed in any one of claims 1 to 21, wherein the genetic construct comprises a structural milk whey protein gene sequence optionally including internal regulatory sequences.

23. A method or process as claimed in any one of claims 1 to 22, wherein the genetic construct comprises a 3' sequence flanking the milk whey protein gene.

24. A method or process as claimed in any one of claims 1 to 23, wherein the genetic construct comprises a cDNA sequence coding for the polypeptide of interest inserted into the first exon of the gene encoding the milk whey protein.

25. A method or process as claimed in any one of claims 1 to 24, wherein the genetic construct includes some of the 5' flanking sequences of the gene encoding the milk whey protein.

26. A method or process as claimed in any one of claims 1 to 25, wherein the genetic construct contains a sequence encoding a signal peptide for the polypeptide of interest.

27. A method or process as claimed in claim 26, wherein the sequence encoding a signal peptide of the milk whey protein gene is precisely fused to that portion of the DNA sequence that encodes the N-terminal amino acid of the mature polypeptide in the substance produced.

28. A method or process as claimed in any one of claims 1 to 27, wherein, the 3' end of the DNA sequence terminates after its stop codon, but before its own polyA addition site.

29. A process for preparing a genetic construct comprising:
(i) a DNA sequence coding for a polypeptide; and
(ii) a DNA sequence from a β-lactoglobulin gene to direct expression of the DNA sequence encoding the polypeptide in the lactating mammary gland of an adult, female, non-human mammal; the process comprising fusing the DNA sequences.

30. A process as claimed in claim 29, comprising any or all of the specific features of claims 19 to 28.

31. A process as claimed in claim 29 or 30, wherein the genetic construct is comprised in a plasmid.

## Patentansprüche (Patentansprüche für folgende(n) Vertragsstaat(en): BE, CH, LI, DE, FR, GB, IT, LU, NL, SE)

1. Verfahren zur Herstellung einer Substanz mit einem Polypeptid in Milch, wobei das Verfahren:
a) die Herstellung von Milch in der Milchdrüse eines erwachsenen weiblichen nicht humanen Säugetiers dessen genetisches Material ein genetisches Konstrukt enthält, das:
(i) eine DNA-Sequenz, die für das Polypeptid kodiert; und
(ii) eine DNA-Sequenz von einem β-Lactoglobulingen zur direkten Expression der DNA-Sequenz, die für das Polypeptid kodiert und
(b) die Sammlung der Milch, die bei Schritt (a) hergestellt wurde, beinhaltet.

2. Verfahren nach Anspruch 1, wobei die Substanz nachher aus der Milch des erwachsenen Säugetieres gewonnen wird.

3. Verfahren nach Anspruch 2, wobei die Substanz aus der Milch nach der post-translatorischen Modifikation gewonnen wird.

4. Verfahren nach Anspruch 1, 2 oder 3, wobei es sich bei der Substanz mit einem Polypeptid um ein Protein handelt.

5. Verfahren nach Anspruch 4, wobei es sich bei der Substanz mit einem Polypeptid um ein humanes Blutprotein handelt.

6. Verfahren nach Anspruch 4, wobei es sich bei der Substanz mit einem Polypeptid um ein Peptidhormon handelt.

7. Verfahren nach Anspruch 5, wobei es sich bei der Substanz mit einem Polypeptid um einen Blutgerinnungsfaktor oder um eine Untereinheit eines Blutgerinnungsfaktors handelt.

8. Verfahren nach einem der Ansprüche 1 bis 4, wobei es sich bei der Substanz mit einem Polypeptid um ein Enzym handelt.

9. Verfahren zur Herstellung einer Substanz in der Milch, die das Reaktionsprodukt eines Enzyms ist, wobei das Verfahren:
(a) die Herstellung von Milch in der Milchdrüse eines erwachsenen weiblichen nicht humanen Säugetiers, dessen genetisches Material ein genetisches Konstrukt enthält, das:
(i) eine DNA-Sequenz, die für das Enzym kodiert enthält; und
(ii) eine DNA-Sequenz von einem Milchmolkeproteingen zur direkten Expression einer DNA-Sequenz, die für das Enzym kodiert; und
(b) die Möglichkeit des Enzyms, die Bildung des Reaktionsprodukts von einem oder mehreren Substraten des Enzyms zu katalysieren, beinhaltet.

10. Verfahren nach Anspruch 9, wobei es sich beim Milchmolkeproteingen um ein β-Lactaglobulingen handelt.

11. Verfahren nach Anspruch 9 oder 10, wobei das Reaktionsprodukt von der Milch des erwachsenen weiblichen Säugetieres gewonnen wird.

12. Verfahren zur Herstellung eines transgenen Säugetieres, wobei das Verfahren:
(a) die In-vitro-Einbindung in ein genetisches Konstrukt mit:
(i) einer DNA-Sequenz, die für ein Polypeptid kodiert und
(ii) einer DNA-Sequenz von einem β-Lactaglobulingen, zur direkten Expression der DNA-Sequenz, die für das Polypeptid in der Milchdrüse eines erwachsenen weiblichen Säugetieres kodiert;
(b) die Einbindung des genetischen Konstrukts in die Keimbahn eines nicht humanen Säugetiers durch Injektion in eine befruchtete Eizelle oder in eine Embryozelle des nicht humanen Säugetiers; und
(c) die Möglichkeit der injizierten befruchteten Eizelle oder des Embryos sich in ein erwachsenes Säugetier zu entwickeln; und
(d) gegebenenfalls die Züchtung eines weiblichen Säugetiers, dessen Keimbahn das genetische Konstrukt des im Schritt (c) hergestellten Säugetiers enthält, beinhaltet.

13. Verfahren nach Anspruch 12, wobei die Injektion in einen Nucleus eines zweizelligen Embryos verabreicht wird.

14. Verfahren nach Anspruch 12, wobei lineare Moleküle der DNA in einen Pro-Nucleus oder einen Nucleus der Eizelle injiziert werden.

15. Verfahren nach einem der Ansprüche 1 bis 11, oder ein Verfahren nach einem der Ansprüche 12, 13 oder 14, wobei es sich beim Säugetier um ein landwirtschaftliches Nutztier handelt.

16. Verfahren, nach Anspruch 15, wobei das Säugetier aus der Familie der *Suidae,* der Gattung *Ovis,* der Gattung *Capra* und der Gattung *Bos* ausgewählt wird.

17. Verfahren nach Anspruch 15, wobei es sich beim Säugetier um ein Milchschaf handelt.

18. Verfahren nach einem der Ansprüche 1 bis 17, wobei es sich beim β-Lactoglobulin um β-Lactoglobulin vom Schaf handelt.

19. Verfahren nach einem der Ansprüche 1 bis 18, wobei das genetische Konstrukt einen Promotor enthält.

20. Verfahren nach einem der Ansprüche 1 bis 19, wobei das genetische Konstrukt eine Startstelle für die Transkription enthält.

21. Verfahren nach einem der Ansprüche 1 bis 20 , wobei das genetische Konstrukt eine oder mehrere distale 5'-Regulatorsequenzen des Milchmolkeproteingens enthält.

22. Verfahren nach einem der Ansprüche 1 bis 21, wobei das genetische Konstrukt eine Strukturgensequenz für das Milchmolkeprotein enthält, wobei gegebenenfalls interne Regulatorsequenzen enthält.

23. Verfahren nach einem der Ansprüche 1 bis 22, wobei das genetische Konstrukt eine 3'-Sequenz enthält, die das Milchmolkeproteingen flankiert.

24. Verfahren nach einem der Ansprüche 1 bis 23, wobei das genetische Konstrukt eine cDNA-Sequenz enthält, die für das im Interesse stehende Polypeptid kodiert, das in das erste Exon des Gens, welches das Milchmolkeprotein kodiert, inseriert wird.

25. Verfahren nach einem der Ansprüche 1 bis 24, wobei das genetische Konstrukt einige der 5'-flankierenden Sequenzen des Gens enthält, welches das Milchmolkeprotein kodiert.

26. Verfahren nach einem der Ansprüche 1 bis 25, wobei das genetische Konstrukt eine Sequenz enthält, die ein Signalpeptid für das im Interesse stehende Polypeptid, kodiert.

27. Verfahren nach Anspruch 26, wobei die Sequenz, die ein Signalpeptid des Milchmolkeproteingens kodiert, präzise zu dem Teil der DNA-Sequenz, die eine N-terminale Aminosäure des reifen Polypeptids in der produzierten Substanz kodiert, fusioniert wird.

28. Verfahren nach einem der Ansprüche 1 bis 27, wobei das 3'-Ende der DNA-Sequenz nach seinem Stopcodon, jedoch vor ihrer eigenen PolyA-Additionsstelle, endet.

29. Genetisches Konstrukt mit:
(i) einer DNA-Sequenz, die für ein Polypeptid kodiert und
(ii) einer DNA-Sequenz eines β-Lactoglobulingens für die direkte Expression der DNA-Sequenz, die das Polypeptid in der laktierenden Milchdrüse eines erwachsenen weiblichen nicht humanen Säugetiers kodiert.

30. Genetisches Konstrukt nach Anspruch 29, wobei einige oder alle speziellen Merkmale der Ansprüche 19 bis 28 beinhaltet sind.

31. Plasmid mit einem genetischen Konstrukt nach den Ansprüchen 29 oder 30.

## Patentansprüche (Patentansprüche für folgende(n) Vertragsstaat(en): AT)

1. Verfahren zur Herstellung einer Substanz mit einem Polypeptid in Milch, wobei das Verfahren:
(a) die Herstellung von Milch in der Milchdrüse eines erwachsenen weiblichen nicht humanen Säugetiers, dessen genetisches Material ein genetisches Konstrukt enthält, das:
(i) eine DNA-Sequenz, die für das Polypeptid kodiert und
(ii) eine DNA-Sequenz von einem β-Lactoglobulingen zur direkten Expression der DNA-Sequenz, die für das Polypeptid kodiert und
(b) die Sammlung der Milch, die in Schritt (a) hergestellt wurde, beinhaltet.

2. Verfahren nach Anspruch 1, wobei die Substanz nachher aus der Milch des erwachsenen weiblichen Säugetiers gewonnen wird.

3. Verfahren nach Anspruch 2, wobei die Substanz aus der Milch nach der transkriptionellen Modifikation gewonnen wird.

4. Verfahren nach Anspruch 1, 2 oder 3, wobei es sich bei der Substanz mit einem Polypeptid um ein Protein handelt.

5. Verfahren nach Anspruch 4, wobei es sich bei der Substanz mit einem Polypeptid um ein humanes Blutprotein handelt.

6. Verfahren nach Anspruch 4, wobei es sich bei der Substanz mit einem Polypeptid um ein Peptidhormon handelt.

7. Verfahren nach Anspruch 5, wobei es sich bei der Substanz mit einem Polypeptid um einen Blutgerinnungsfaktor oder eine Untereinheit eines Blutgerinnungsfaktors handelt.

8. Verfahren nach einem der Ansprüche 1 bis 4, wobei es sich bei der Substanz mit einem Polypeptid um ein Enzym handelt.

9. Verfahren zur Herstellung einer Substanz in der Milch, die das Reaktionsprodukt eines Enzyms ist, wobei das Verfahren:
(a) die Herstellung von Milch in der Milchdrüse eines erwachsenen weiblichen nicht humanen Säugetieres, dessen genetisches Material ein genetisches Konstrukt enthält, das:
(i) eine DNA-Sequenz, die für das Enzym kodiert enthält und
(ii) eine DNA-Sequenz eines Milchmolkeproteingens zur direkten Expression der DNA-Sequenz, die für das Enzym kodiert und
(b) die Möglichkeit des Enzyms, die Bildung des Reaktionsprodukts von einem oder mehreren Substraten des Enzyms zu katalysieren, beinhaltet.

10. Verfahren nach Anspruch 9, wobei es sich beim Milchmolkeproteingen um ein β-Lactoglobulingen handelt.

11. Verfahren nach Anspruch 9 oder 10, wobei das Reaktionsprodukt aus der Milch des erwachsenen weiblichen Säugetiers gewonnen wird.

12. Verfahren für die Herstellung eines transgenen Säugetieres, wobei das Verfahren:
(a) die in-vitro-Einbindung in ein genetisches Konstrukt mit:
(i) einer DNA-Sequenz, die für ein Polypeptid kodiert und
(ii) einer DNA-Sequenz von einem β-Lactoglobulin zur direkten Expression der DNA-Sequenz, die von einem Polypeptid in der Milchdrüse eines erwachsenen weiblichen Säugetiers kodiert
(b) die Einbindung des genetischen Konstrukts in die Keimbahn eines nicht humanen Säugetiers durch Injektion in eine befruchtete Eizelle oder einer Embryozelle des nicht humanen Säugetiers und
(c) die Möglichkeit der befruchteten Eizelle oder des Embryos, sich in ein erwachsenes Säugetier zu entwickeln und
(d) gegebenenfalls die Züchtung eines weiblichen Säugetiers, dessen Keimbahn das genetische Konstrukt des Säugetiers in Schritt (c) enthält, beinhaltet.

13. Verfahren nach Anspruch 12, wobei die Injektion in den Nucleus eines zweizelligen Embryos durchgeführt wird.

14. Verfahren nach Anspruch 12, wobei lineare Moleküle der DNA in einen Pro-Nucleus oder einen Nucleus der Eizelle injiziert werden.

15. Verfahren nach einem der Ansprüche 1 bis 11, oder ein Verfahren nach den Ansprüchen 12, 13 oder 14, wobei es sich beim Säugetier um ein Landwirtschaftliches Nutztier handelt.

16. Verfahren nach Anspruch 15, wobei das Säugetier aus der Familie der *Suidae,* der Gattung der *Ovis,* der Gattung *Capra* und der Gattung *Bos,* ausgewählt wird.

17. Verfahren nach Anspruch 15, wobei es sich beim Säugetier um ein Milchschaf handelt.

18. Verfahren nach einem der Ansprüche 1 bis 17, wobei es sich beim β-Lactoglobulin um β-Lactoglobulin vom Schaf handelt.

19. Verfahren nach einem der Ansprüche 1 bis 18, wobei das genetische Konstrukt einen Promotor enthält.

20. Verfahren nach einem der Ansprüche 1 bis 19, wobei das genetische Konstrukt eine Startsequenz für die Transkription enthält.

21. Verfahren nach einem der Ansprüche 1 bis 20, wobei das genetische Konstrukt eine oder mehrere distale 5'-Regulatorsequenzen des Milchmolkeproteingens enthält.

22. Verfahren nach einem der Ansprüche 1 bis 21, wobei das genetische Konstrukt eine Sequenz für das Milchmolkestrukturgen und gegebenenfalls eine interne Regulatorsequenz enthält.

23. Verfahren nach einem der Ansprüche 1 bis 22, wobei das genetische Konstrukt eine 3'-Sequenz enthält, die das Milchmolkeproteingen flankiert.

24. Verfahren nach einem der Ansprüche 1 bis 23, wobei das genetische Konstrukt eine cDNA-Sequenz enthält, die für das im Interesse stehende Polypeptid kodiert, das in das erste Exon des Gens inseriert wurde, welches für das Milchmolkeprotein kodiert.

25. Verfahren nach einem der Ansprüche 1 bis 24, wobei das genetische Konstrukt einige der 5'-flankierenden Sequenzen des Gens enthält, welches das Milchmolkeprotein kodiert.

26. Verfahren nach einem der Ansprüche 1 bis 25, wobei das genetische Konstrukt eine Sequenz enthält, die ein Signalpeptid für das im Interesse stehende Polypeptid kodiert.

27. Verfahren nach Anspruch 26, wobei die Sequenz, die ein Signalpeptid des Milchmolkeproteingens kodiert, präzise zum Teil der DNA-Sequenz, die die N-terminale Aminosäure des reifen Polypeptids in der hergestellten Substanz kodiert, fusioniert wird.

28. Verfahren nach einem der Ansprüche 1 bis 27, wobei das 3'-Ende der DNA-Sequenz nach seinem Stopcodon, aber noch vor seiner eigenen PolyA-Additionsstelle endet.

29. Verfahren zur Herstellung eines genetischen Konstrukts mit:
(i) einer DNA-Sequenz, die für ein Polypeptid kodiert und
(ii) einer DNA-Sequenz eines β-Lactoglobulingens zur direkten Expression der DNA-Sequenz, die das Polypeptid in der laktierenden Milchdrüse eines erwachsenen weiblichen nicht humanen Säugetiers kodiert, wobei das Verfahren die Fusion der DNA-Sequenzen beinhaltet.

30. Verfahren nach Anspruch 29, wobei einige oder alle speziellen Merkmale der Ansprüche 19 bis 28 beinhaltet sind.

31. Verfahren nach den Ansprüchen 29 oder 30, wobei das genetische Konstrukt in einem Plasmid enthalten ist.

## Revendications (Revendications pour l'(les) Etat(s) contractant(s) suivant(s): BE, CH, LI, DE, FR, GB, IT, LU, NL, SE)

1. Une méthode de production dans le lait d'une substance comprenant un polypeptide, la méthode comprenant :
(a) la production de lait dans la glande mammaire d'un animal mammifère non humain adulte, femelle, dont le matériel génétique contient une construction génétique qui comprend:
(i) Une séquence d'ADN codant pour le polypeptide; et
(ii) une séquence d'ADN provenant d'un gène de la β-lactoglobuline pour diriger l'expression de la séquence d'ADN codant pour le polypeptide; et
(b) la récolte du lait produit dans l'étape (a).

2. Une méthode selon la revendication 1, dans laquelle la substance est subséquemment recueillie du lait du mammifère adulte femelle.

3. Une méthode selon la revendication 2, dans laquelle la substance est recueillie du lait après modification post-traductionelle.

4. Une méthode selon la revendication 1, 2, ou 3, dans laquelle la substance comprenant un polypeptide est une protéine.

5. Une méthode selon la revendication 4, dans laquelle la substance comprenant un polypeptide est une protéine sanguine humaine.

6. Une méthode selon la revendication 4, dans laquelle la substance comprenant un polypeptide est une hormone peptidique.

7. Une méthode selon la revendication 5, dans laquelle la substance comprenant un polypeptide est un facteur de coagulation sanguine ou une sous-unité d'un facteur de coagulation sanguine.

8. Une méthode selon l'une des revendications 14, dans laquelle la substance comprenant un polypeptide est une enzyme.

9. Une méthode de production dans le lait d'une substance qui est le produit de réaction d'une enzyme, la méthode comprenant:
(a) la production de lait dans la glande mammaire d'un animal mammifère non humain adulte, femelle, dont le matériel génétique contient une construction génétique qui comprend:
(i) Une séquence d'ADN codant pour l'enzyme; et
(ii) une séquence d'ADN provenant d'un gène d'une protéine du petit lait pour diriger l'expression de la séquence d'ADN codant pour l'enzyme; et
(b) la possibilité pour l'enzyme de catalyser la formation du produit de réaction à partir d'un ou de plusieurs substrats de l'enzyme.

10. Une méthode selon la revendication 9, dans laquelle le gène de la protéine de petit lait est le gène de la β-lactoglobuline.

11. Une méthode selon la revendication 9 ou 10, dans laquelle le produit de réaction est recueilli du lait du mammifère adulte femelle.

12. Un procédé pour la production d'un mammifère transgénique, le procédé comprenant:
(a) l'incorporation *in vitro* d'une construction génétique comprenant:
(i) une séquence d'ADN codant pour un polypeptide; et
(ii) une séquence d'ADN provenant d'un gène de la β-lactoglobuline pour diriger l'expression de la séquence d'ADN codant pour le polypeptide dans la glande mammaire d'un mammifère adulte femelle; et
(b) l'incorporation de la construction génétique dans la lignée germinale d'un mammifère non humain par injection dans un oeuf fertilisé ou dans une cellule de l'embryon du mammifère non humain; et
(c) la possibilité pour l'oeuf fertilisé ou l'embryon de se développer en mammifère adulte; et
(d) éventuellement, l'élevage d'un mammifère femelle, dont la lignée germinale contient la construction génétique provenant du mammifère produit dans l'étape (c).

13. Un procédé selon la revendication 12, dans lequel l'injection est effectuée dans le noyau d'un embryon bi-cellulaire.

14. Un procédé selon la revendication 12, dans lequel des molécules linéaires d'ADN sont injectées dans le noyau ou le pro-noyau de l'oeuf.

15. Une méthode selon l'une des revendications 1 à 11, ou un procédé selon les revendications 12, 13 ou 14, dans lequel le mammifère est un mammifère d'élevage domestiqué.

16. Une méthode ou un procédé selon la revendication 15, dans lequel le mammifère est choisi parmi les membres de la famille Suidae, les membres du genre Ovis, les membres du genre Capra et les membres du genre Bos.

17. Une méthode ou un procédé selon la revendication 15, dans lequel le mammifère est une brebis laitière.

18. Une méthode ou un procédé selon l'une des revendications 1 à 17, dans lequel la β-lactoglobuline est la β-lactoglobuline ovine.

19. Une méthode ou un procédé selon l'une des revendications 1 à 18, dans lequel la construction génétique comprend un promoteur.

20. Une méthode ou un procédé selon l'une des revendications 1 à 19, dans lequel la construction génétique comprend un site d'initiation de la transcription.

21. Une méthode ou un procédé selon l'une des revendications 1 à 20, dans lequel la construction génétique comprend une ou plusieurs séquences distales 5' régulatrices du gène de la protéine du petit lait.

22. Une méthode ou un procédé selon l'une des revendications 1 à 21, dans lequel la construction génétique comprend une séquence d'un gène de structure d'une protéine du petit lait, comprenant éventuellement des séquences intemes de régulation.

23. Une méthode ou un procédé selon l'une des revendications 1 à 22, dans lequel la construction génétique comprend une séquence 3' flanquant le gène de la protéine du petit lait.

24. Une méthode ou un procédé selon l'une des revendications 1 à 23, dans lequel la construction génétique comprend une séquence d'ADNc codant pour le polypeptide d'intérêt insérée dans le premier exon du gène codant pour la protéine du petit lait.

25. Une méthode ou un procédé selon l'une des revendications 1 à 24, dans lequel la construction génétique comprend une partie des séquences 5' flanquantes du gène codant pour la protéine du petit lait.

26. Une méthode ou un procédé selon l'une des revendications 1 à 25, dans lequel la construction génétique comprend une séquence codant pour un peptide signal du polypeptide d'intérêt.

27. Une méthode ou un procédé selon la revendication 26, dans lequel la séquence codant pour le peptide signal du gène de la protéine du petit lait est fusionnée précisément à la portion de la séquence d'ADN qui code pour l'acide aminé N-terminal du polypeptide mature dans la substance produite.

28. Une méthode ou un procédé selon l'une des revendications 1 à 27, dans lequel l'extrémité 3' de la séquence d'ADN se termine après son codon stop, mais avant son propre site d'addition de polyA.

29. Une construction génétique comprenant:
(i) une séquence d'ADN codant pour un polypeptide; et
(ii) une séquence d'ADN provenant d'un gène de la β-lactoglobuline pour diriger l'expression de la séquence d'ADN codant pour le polypeptide dans la glande mammaire lactifère d'un mammifère non humain, adulte et femelle.

30. Une construction génétique selon la revendication 29, comprenant une ou toutes les caractéristiques spécifiques des revendications 19 à 28.

31. Un plasmide comprenant une construction génétique selon la revendication 29 ou 30.

## Revendications (Revendications pour l'(les) Etat(s) contractant(s) suivant(s): AT)

1. Une méthode de production dans le lait d'une substance comprenant un polypeptide, la méthode comprenant :
(a) la production de lait dans la glande mammaire d'un animal mammifère non humain adulte, femelle, dont le matériel génétique contient une construction génétique qui comprend:
(i) Une séquence d'ADN codant pour le polypeptide; et
(ii) une séquence d'ADN provenant d'un gène de la β-lactoglobuline pour diriger l'expression de la séquence d'ADN codant pour le polypeptide; et
(b) la récolte du lait produit dans l'étape (a).

2. Une méthode selon la revendication 1, dans laquelle la substance est subséquemment recueillie du lait du mammifère adulte femelle.

3. Une méthode selon la revendication 2, dans laquelle la substance est recueillie du lait après modification post-transcriptionelle.

4. Une méthode selon la revendication 1, 2, ou 3, dans laquelle la substance comprenant un polypeptide est une protéine.

5. Une méthode selon la revendication 4, dans laquelle la substance comprenant un polypeptide est une protéine sanguine humaine.

6. Une méthode selon la revendication 4, dans laquelle la substance comprenant un polypeptide est une hormone peptidique.

7. Une méthode selon la revendication 5, dans laquelle la substance comprenant un polypeptide est un facteur de coagulation sanguine ou une sous-unité d'un facteur de coagulation sanguine.

8. Une méthode selon l'une des revendications 1-4, dans laquelle la substance comprenant un polypeptide est une enzyme.

9. Une méthode de production dans le lait d'une substance qui est le produit de réaction d'une enzyme, la méthode comprenant:
(a) la production de lait dans la glande mammaire d'un animal mammifère non humain adulte, femelle, dont le matériel génétique contient une construction génétique qui comprend:
(i) Une séquence d'ADN codant pour l'enzyme; et
(ii) une séquence d'ADN provenant d'un gène d'une protéine du petit lait pour diriger l'expression de la séquence d'ADN codant pour l'enzyme; et
(b) la possibilité pour l'enzyme de catalyser la formation du produit de réaction à partir d'un ou de plusieurs substrats de l'enzyme.

10. Une méthode selon la revendication 9, dans laquelle le gène de la protéine de petit lait est le gène de la β-lactoglobuline.

11. Une méthode selon la revendication 9 ou 10, dans laquelle le produit de réaction est recueilli du lait du mammifère adulte femelle.

12. Un procédé pour la production d'un mammifère transgénique, le procédé comprenant:
(a) l'incorporation *in vitro* d'une construction génétique comprenant:
(i) une séquence d'ADN codant pour un polypeptide; et
(ii) une séquence d'ADN provenant d'un gène de la β-lactoglobuline pour diriger l'expression de la séquence d'ADN codant pour le polypeptide dans la glande mammaire d'un mammifère adulte femelle; et
(b) l'incorporation de la construction génétique dans la lignée germinale d'un mammifère non humain par injection dans un oeuf fertilisé ou dans une cellule de l'embryon du mammifère non humain; et
(c) la possibilité pour l'oeuf fertilisé ou l'embryon de se développer en mammifère adulte; et
(d) éventuellement, l'élevage d'un mammifère femelle, dont la lignée germinale contient la construction génétique provenant du mammifère produit dans l'étape (c).

13. Un procédé selon la revendication 12, dans lequel l'injection est effectuée dans le noyau d'un embryon bi-cellulaire.

14. Un procédé selon la revendication 12, dans lequel des molécules linéaires d'ADN sont injectées dans le noyau ou le pro-noyau de l'oeuf.

15. Une méthode selon l'une des revendications 1 à 11, ou un procédé selon les revendications 12, 13 ou 14, dans lequel le mammifère est un mammifère d'élevage domestiqué.

16. Une méthode ou un procédé selon la revendication 15, dans lequel le mammifère est sélectionné parmi les membres de la famille Suidae, les membres du genre Ovis, les membres du genre Capra et les membres du genre Bos.

17. Une méthode ou un procédé selon la revendication 15, dans lequel le mammifère est une brebis laitière.

18. Une méthode ou un procédé selon l'une des revendications 1 à 17, dans lequel la β-lactoglobuline est la β-lactoglobuline ovine.

19. Une méthode ou un procédé selon l'une des revendications 1 à 18, dans lequel la construction génétique comprend un promoteur.

20. Une méthode ou un procédé selon l'une des revendications 1 à 19, dans lequel la construction génétique comprend un site d'initiation de la transcription.

21. Une méthode ou un procédé selon l'une des revendications 1 à 20, dans lequel la construction génétique comprend une ou plusieurs séquences distales 5' régulatrices du gène de la protéine du petit lait.

22. Une méthode ou un procédé selon l'une des revendications 1 à 21, dans lequel la construction génétique comprend une séquence d'un gène de structure d'une protéine du petit lait, comprenant éventuellement des séquences internes de régulation.

23. Une méthode ou un procédé selon l'une des revendications 1 à 22, dans lequel la construction génétique comprend une séquence 3' flanquant le gène de la protéine du petit lait.

24. Une méthode ou un procédé selon l'une des revendications 1 à 23, dans lequel la construction génétique comprend une séquence d'ADNc codant pour le polypeptide d'intérêt insérée dans le premier exon du gène codant pour la protéine du petit lait.

25. Une méthode ou un procédé selon l'une des revendications 1 à 24, dans lequel la construction génétique comprend une partie des séquences 5' flanquantes du gène codant pour la protéine du petit lait.

26. Une méthode ou un procédé selon l'une des revendications 1 à 25, dans lequel la construction génétique comprend une séquence codant pour un peptide signal du polypeptide d'intérêt.

27. Une méthode ou un procédé selon la revendication 26, dans lequel la séquence codant pour le peptide signal du gène de la protéine du petit lait est fusionnée précisément à la portion de la séquence d'ADN qui code l'acide aminé N-terminal du polypeptide mature dans la substance produite.

28. Une méthode ou un procédé selon l'une des revendications 1 à 27, dans lequel l'extrémité 3' de la séquence d'ADN se termine après son codon

29. Un procédé pour préparer une construction génétique comprenant:
(i) une séquence d'ADN codant pour un polypeptide; et
(ii) une séquence d'ADN provenant d'un gène de la β-lactoglobuline pour diriger l'expression de la séquence d'ADN codant pour le polypeptide dans la glande mammaire lactifère d'un mammifère non humain, adulte et femelle. Le procédé comprenant le fusionnement des séquences d'ADN.

30. Un procédé selon la revendication 29, comprenant une ou toutes les caractéristiques spécifiques des revendications 19 à 28.

31. Un procédé selon la revendication 29 ou 30, dans lequel la construction génétique est comprise dans un plasmide.
